# EUROPEAN PATENT APPLICATION

(11) **EP 4 549 465 A1**
(43) Date of publication of application: **07.05.2025**
(21) Application number: 23830423.2
(22) Date of filing: 29.06.2023
(51) Int. Cl.: C07K 16/28, A61K 39/395, A61P 35/00

(54) **MUTANT OF ANTIBODY VARIABLE REGION, AND USE THEREOF**

(30) Priority: 29.06.2022 CN 202210763046
(71) Applicant: Shenyang Pharmaceutical University, Shenyang, Liaoning 110016 (CN); Beijing Sipuruige Biotech Corporation, Beijing 100176 (CN)
(72) Inventor: MA, Ningning, Benxi, Liaoning 117000 (CN); LI, Mingying, Benxi, Liaoning 117000 (CN); YU, Yue, Benxi, Liaoning 117000 (CN); LIU, Sixu, Benxi, Liaoning 117000 (CN); ZHANG, Nan, Benxi, Liaoning 117000 (CN); LI, Jiayun, Shijiazhuang, Hebei 050000 (CN); XU, Weiwei, Beijing 102600 (CN); ZHENG, Bobo, Tianjin 300180 (CN)
(74) Representative: Altmann Stößel Dick Patentanwälte PartG mbB
(86) International application number: PCT/CN2023/103976
(87) International publication number: WO 2024/002258

(57) **Abstract**

The present disclosure provides a mutant of an antibody, and use thereof. Specifically, according to the Kabat numbering system, the mutant of the antibody has an engineered cysteine residue at any one or more position(s) selected from: positions 12, 34, 35, 38, 44, 47, 51, 60, 61, 67, 69, 78, 79, and 114 of the heavy chain variable region, or any combination thereof; or positions 19, 21, 44, 46, 47, 48, 62, 71, 75, 78, and 87 of the light chain variable region, or any combination thereof. The sulfhydryl group on the engineered cysteine can remain partially active in the antibody expression process. The active sulfhydryl group can be directly used to react with other active groups without reduction treatment. The cysteine-engineered antibody of the present disclosure can obtain the active groups for conjugating a drug, which is conducive to simplifying the production process of the antibody-drug conjugates, improving the homogeneity of the antibody-drug conjugates, increasing the efficacy, and reducing the toxicity and side effects.

## Description

### TECHNICAL FIELD

The present disclosure relates to the field of antibodies. Specifically, it relates to the cysteine mutants of antibodies, compositions and/or conjugates comprising the same, and uses thereof.

### BACKGROUND

Antibody-drug conjugates (ADCs) are antibody products that are connected with drug molecules, and obtained by linking the antibodies to the drugs via biological or chemical means. Antibody-drug conjugates reduce the killing effect of the drug on normal cells through the specificity of monoclonal antibodies, and are mainly used in the treatment of tumors. Antibody-drug conjugates are mainly prepared through the conjugated reaction of the amino groups of lysine or the sulfhydryl groups of cysteine. Traditional non-site-specific conjugation methods, whether by lysine residue conjugation or cysteine residue conjugation, have some drawbacks. Site-specific conjugation through the site-specific conjugation technology can improve the homogeneity of antibody-drug conjugates ^{[1]}.

Antibody molecules comprise four polypeptide chains, of which the two chains with higher molecular weights are called heavy chains (HCs), while the two chains with lower molecular weights are called light chains (LCs). In the same antibody molecule, the amino acid compositions of the two H-chains are identical and the amino acid compositions of the two L-chains are identical. By analyzing the amino acid sequences of the heavy and light chains of different antibodies, it was found that the amino acid sequences near the N-terminal ends of the heavy and light chains varied significantly, while the amino acid sequences of the rest of the chains are relatively constant. Therefore, the regions with significant variations in the amino acid sequences near the N-terminal ends of the heavy and light chains of the antibody are called variable regions (Vs), accounting for 1/4 and 1/2 of the heavy and light chains, respectively; and the regions with relatively stable amino acid sequences near the C-terminal ends are called constant regions (Cs), accounting for 3/4 and 1/2 of the heavy and light chains, respectively.

A single chain antibody fragment (scFv) is an antibody formed by linking the heavy chain variable region of an antibody to the light chain variable region the antibody by a short amino acid peptide (such as a linker). A single chain antibody fragment (scFv) is an antibody in which the heavy chain variable region and the light chain variable region are spaced by a short amino acid sequence, and connected by disulfide bonds ^{[2]}. ScFv has a low molecular weight, is suitable to be expressed in yeast or bacteria, can be produced rapidly in large quantities and is an ideal candidate for high-throughput selection techniques such as phage display, cell display, yeast display, and ribosome display ^{[3]}. Single chain antibody fragment-drug conjugates have great potential. Single chain antibody fragment-drug conjugates are more potent and have low side effects due to the shorter half-life of scFv as well as its tumor penetration capacity that appears to be greater than that of the Fab fragment ^{[4]}. Data demonstrate that single chain antibody fragment-drug conjugates are better tolerated than IgG antibody-drug conjugates ^{[5, 6]}.

The site-specific conjugation technique is not only applicable to intact IgG antibody molecules, but also applicable to a scFv and the scFv-based bispecific antibody-drug conjugates. The most common reactive amino acid in the site-specific conjugation technique is cysteine. Antibody-drug conjugates with the controlled drug-antibody conjugation ratios can be developed by the site-specific mutagenesis technique. Selection of appropriate sites for cysteine mutation allows the sulfhydryl groups on cysteine obtained by mutation to remain free throughout the expression and purification process and to be directly applied to the downstream conjugated reaction. Due to the widespread use of cysteine, maleimide is the most popular linker group ^{[7]}. An antibody is then conjugated to another functional molecule (such as an antibody fragment, a peptide, or a small-molecule drug) via a linker containing maleimide.

Most of the sulfhydryl groups on cysteine that are introduced into the antibodies through cysteine engineering can be oxidized during cell culture, such as by reacting with the sulfhydryl groups on free cysteine in the cell culture medium to form disulfide bonds. The oxidized sulfhydryl groups need to be reduced to be active for subsequent conjugated reactions. However, the reduction reaction will open the disulfide bonds in the antibodies at the same time, and these opened disulfide bonds in the antibodies need to be re-oxidized to restore the disulfide bond structure in the antibodies. The series of reprocessing steps following the expression of these antibodies can complicate the production process of the antibody-drug conjugates and pose challenges for the quality control.

### SUMMARY OF THE INVENTION

The present disclosure mainly provides a cysteine-engineered antibody, according to the Kabat numbering system, having an engineered cysteine residue at any one or more positions selected from: positions 12, 34, 35, 38, 44, 47, 51, 60, 61, 67, 69, 78, 79, and 114 of the heavy chain variable region or any combination thereof, which correspond to the IMGT numbers of 13, 39, 40, 43, 49, 52, 56, 67, 68, 75, 77, 86, 87, and 122, respectively; or positions 19, 21, 44, 46, 47, 48, 62, 71, 75, 78, and 87 of the light chain variable region or any combination thereof, which correspond to the IMGT numbers of 19, 21, 50, 52, 53, 54, 76, 87, 91, 94, and 103, respectively. The sulfhydryl group on the engineered cysteine can remain partially active in the antibody expression process. The active sulfhydryl group can be directly used to react with other active groups without reduction treatment.

Specifically, the present disclosure provides the following aspects:
1. A cysteine-engineered antibody, wherein, according to the Kabat numbering system, the antibody has an engineered cysteine residue at any one or more position(s) selected from:
   positions 12, 34, 35, 38, 44, 47, 51, 60, 61, 67, 69, 78, 79, and 114 of the heavy chain variable region, or any combination thereof; or
   positions 19, 21, 44, 46, 47, 48, 62, 71, 75, 78, and 87 of the light chain variable region, or any combination thereof.
2. The cysteine-engineered antibody according to item 1, wherein, according to the Kabat numbering system, the antibody has an engineered cysteine residue at any one or more position(s) selected from:
   positions 12, 35, 61, 51, 69, and 78 of the heavy chain variable region, or any combination thereof; or
   positions 46, and 87 of the light chain variable region, or any combination thereof.
3. The cysteine-engineered antibody according to item 1, wherein, according to the Kabat numbering system, the antibody has an engineered cysteine residue at any one or more positions selected from:
   positions 12, 35, 61, and 51 of the heavy chain variable region, or any combination thereof; or
   positions 46, and 87 of the light chain variable region, or any combination thereof.
4. The cysteine-engineered antibody according to item 1, wherein, the cysteine-engineered antibody is derived from the following wild-type antibody or antibodies:
   an antibody comprising the heavy chain variable region as set forth in SEQ ID NO: 4 and the light chain variable region as set forth in SEQ ID NO: 5;
   an antibody comprising the heavy chain variable region as set forth in SEQ ID NO: 75 and the light chain variable region as set forth in SEQ ID NO: 92;
   an antibody comprising the heavy chain variable region as set forth in SEQ ID NO: 81 and the light chain variable region as set forth in SEQ ID NO: 95;
   an antibody comprising the heavy chain variable region as set forth in SEQ ID NO: 85 and the light chain variable region as set forth in SEQ ID NO: 96;
   an antibody comprising the heavy chain variable region as set forth in SEQ ID NO: 89 and the light chain variable region as set forth in SEQ ID NO: 97;
   an antibody comprising the heavy chain as set forth in SEQ ID NO: 100 and the light chain as set forth in SEQ ID NO: 101;
   an antibody comprising the heavy chain variable region as set forth in SEQ ID NO: 108 and the light chain variable region as set forth in SEQ ID NO: 109;
   an antibody comprising the heavy chain variable region as set forth in SEQ ID NO: 113 and the light chain variable region as set forth in SEQ ID NO: 114; and/or
   an antibody comprising the heavy chain variable region as set forth in SEQ ID NO: 130 and the light chain variable region as set forth in SEQ ID NO: 133.
5. The cysteine-engineered antibody according to item 1, wherein, the light chain of the antibody is type λ or κ;
   optionally, the antibody is an anti-HER2 antibody, an anti-CD3 antibody, an anti-CD20 antibody, an anti-VEGFR-2 antibody, an anti-EGFR antibody, and/or an anti-c-Met antibody;
   optionally, the antibody is a single chain antibody fragment, an IgG antibody, or a bispecific antibody such as a bispecific antibody in the form of BiTE/DART/Diabody.
6. The cysteine-engineered antibody according to item 5, wherein, when the antibody is a single chain antibody fragment, the antibody further comprises a linking chain, preferably a linking chain of SEQ ID NO. 6.
7. A conjugate comprising the cysteine-engineered antibody according to any one of items 1-6, and a conjugated moiety.
8. The conjugate according to item 7, wherein,
   the conjugated moiety is selected from: polyethylene glycol, a cytotoxic agent, an active peptide, a nanobody, a single domain antibody, a Fab fragment, a Fab' fragment, scFv, a small molecule drug (*e.g.*, a topoenzyme inhibitor, tubulysin A, DM1, PBD, MMAE, or MMAF, etc.), a chemotherapeutic agent, or a radiotherapeutic agent;
   the conjugated moiety is conjugated to the cysteine-engineered antibody via a linker;
   preferably, the linker comprises an electrophilic group (preferably a maleimide group or a haloacetamide group); optionally, the linker is mc-VC-PAB.
9. The conjugate according to item 8, wherein, the polyethylene glycol is a Methoxy PEG Maleimide, preferably mPEG2000-Mal, mPEG5000-Mal, or mPEG10000-Mal.
10. A pharmaceutical composition comprising the cysteine-engineered antibody according to any one of items 1-6 or the conjugate according to any one of items 7-9, and optionally, a pharmaceutical carrier.
11. Use of the cysteine-engineered antibody according to any one of items 1-6, the conjugate according to any one of items 7-9, or the pharmaceutical composition according to item 10 in the manufacture of a medicament or a kit for the treatment of a cancer (*e.g.*, a HER2-positive cancer, preferably breast cancer).
12. A kit comprising the cysteine-engineered antibody according to any one of items 1-6, the conjugate according to any one of items 7-9, or the pharmaceutical composition according to item 10.

### TECHNICAL EFFECTS OF THE PRESENT INVENTION

1. An active group that can be used to conjugate a drug is obtained by utilizing cysteine mutation, which is widely used and facilitates the conjugation.
2. The site-specific mutation can improve the homogeneity of the antibody-drug conjugates as compared to non-site-specific mutation.
3. The selected cysteine mutation sites can maintain the activity of some of the sulfhydryl groups after the expression of the antibody or antibody fragment, and thus can be used in the conjugated reaction without treatment.
4. The site-specific cysteine mutations herein do not change the activity of the antibody, and can increase the efficacy of the drug and reduce the toxic side effects after conjugating to a drug.
5. The mutation sites in the present disclosure are applicable to most of the antibodies and have a certain degree of versatility.
6. The present disclosure can utilize different antibodies to carry out site mutation to construct bispecific antibody-drug conjugates.

### DESCRIPTION OF THE DRAWINGS

Figure 1 shows the SDS-PAGE electropherograms of the purified wild-type anti-HER2 Trastuzumab single chain antibody fragment (WT) and the mutants of single-chain antibody fragments (HC: 12C, 35C, 61C, 34C, 38C, 44C, 47C, 51C, 60C, 67C, 68C, 78C, 79C and 114C; LC:21C, 47C, 48C, 71C, 75C, 19C, 46C, 62C, 78C and 87C). As can be seen from the electrophoretograms, the antibody bands are single, and the purity meets the needs of subsequent experiments.
Figure 2 shows the SDS-PAGE electropherograms of the mutant LC46C of the anti-HER2 Trastuzumab single chain antibody fragment with a mutation in the light chain variable region and the mutant HC12C of the anti-HER2 Trastuzumab single chain antibody fragment with a mutation in the heavy-chain variable region after conjugated with mPEG2000-MAL. As can be seen from the figure, the mutant LC46C with a mutation in the light chain variable region and the mutant HC12C with a mutation in the heavy chain variable region can be successfully conjugated with the mPEG2000-MAL.
Figure 3 shows the SDS-PAGE electropherogram of the mutant LC62C of the anti-HER2 Trastuzumab single chain antibody fragment with a mutation in the light chain variable region after conjugated with mPEG2000-MAL. As can be seen from the figure, the mutant LC62C with a mutation in the light chain variable region can be successfully conjugated with the mPEG2000-MAL.
Figure 4 shows the SDS-PAGE electropherograms of the mutant LC87C of the anti-HER2 Trastuzumab single chain antibody fragment, and the mutants HC38C, HC67C, and HC35C of the anti-HER2 Trastuzumab single chain antibody fragment after conjugated with mPEG2000-MAL. As can be seen from the figure, the mutant LC87C, and the mutants HC38C, HC67C, and HC35C can be successfully conjugated with the mPEG2000-MAL.
Figure 5 shows the SDS-PAGE electropherogram of the mutant HC78C of the anti-HER2 Trastuzumab single chain antibody fragment after conjugated with mPEG2000-MAL. As can be seen from the figure, the mutant HC78C can be successfully conjugated with mPEG2000-MAL.
Figure 6 shows the SDS-PAGE electropherogram of the mutant HC61C of the anti-HER2 Trastuzumab single chain antibody fragment after conjugated with mPEG2000-MAL. As can be seen from the figure, the mutant HC61C can be successfully conjugated with mPEG2000-MAL.
Figure 7 shows the SDS-PAGE electropherogram of the mutants HC69C and HC51C of the anti-HER2 Trastuzumab single chain antibody fragment and the mutant LC71C of the anti-HER2 Trastuzumab single chain antibody fragment after conjugated with mPEG2000-MAL. As can be seen from the figure, the mutants HC69C and HC51C and the mutant LC71C can be successfully conjugated with mPEG2000-MAL.
Figure 8 shows the SDS-PAGE electropherogram of the mutant LC48C of the anti-HER2 Trastuzumab single chain antibody fragment after conjugated with mPEG2000-MAL. As can be seen from the figure, the mutant LC48C can be successfully conjugated with mPEG2000-MAL.
Figure 9 shows the molecular weight deconvolution result of the mutant LC87C of the anti-HER2 Trastuzumab single chain antibody fragment detected by the mass spectrometry. As can be seen from the figure, the molecular weight of the mutant LC87C is 26521.3Da.
Figure 10 shows the molecular weight deconvolution result of the mutant LC87C of the anti-HER2 Trastuzumab single chain antibody fragment conjugated with vcMMAE detected by the mass spectrometry. As can be seen from the figure, the molecular weight of the mutant after conjugation is 27838.2 Da.
Figure 11 shows the molecular weight deconvolution result of the two-site cysteine mutant of the anti-HER2 Trastuzumab single chain antibody fragment detected by the mass spectrometry. As can be seen from the figure, the molecular weight of the mutant is 26553.5 Da.
Figure 12 shows the molecular weight deconvolution result of the two-site cysteine mutant of the anti-HER2 Trastuzumab single chain antibody fragment conjugated with vcMMAE detected by the mass spectrometry. As can be seen from the figure, the molecular weights of the mutant after conjugation are mainly 27988.0 Da and 29186.3 Da.
Figure 13 shows the molecular weight deconvolution result of the two-site mutant of bispecific antibody detected by the mass spectrometry. As can be seen from the figure, the molecular weight of the mutant is 52588.5Da.
Figure 14 shows the molecular weight deconvolution result of the two-site mutant of bispecific antibody conjugated with vcMMAE detected by the mass spectrometry. As can be seen from the figure, the molecular weights of the mutant after conjugation are mainly 53903.7 Da and 55220.6 Da.
Figure 15 shows the SDS-PAGE electropherogram of the mutants LC46C and LC87C of the anti-HER2 Pertuzumab single chain antibody fragment after conjugated with mPEG2000-MAL. As can be seen from the figure, the mutants LC46C and LC87C can be successfully conjugated with mPEG2000-MAL.
Figure 16 shows the SDS-PAGE electropherogram of the mutant LC46C of the anti-CD20 Rituximab single chain antibody fragment and the mutant LC46C of the anti-CD3 Muromonab single chain antibody fragment after conjugated with mPEG2000-MAL. As can be seen from the figure, both the mutants LC46C of the Rituximab single chain antibody fragment and of the Muromonab single chain antibody fragment can be successfully conjugated with mPEG2000-MAL.
Figure 17 shows the SDS-PAGE electropherogram of the mutant LC87C of the anti-VEGFR-2 Ramucirumab single chain antibody fragment after conjugated with mPEG2000-MAL. As can be seen from the figure, the mutant LC87C of the Ramucirumab single chain antibody fragment can be successfully conjugated with mPEG2000.
Figure 18 shows the SDS-PAGE electropherogram of the mutants 46C and 87C of the Trastuzumab with mutations in the light chain variable region after conjugated with mPEG2000-MAL. As can be seen from the figure, the mutants 46C and 87C of the Trastuzumab can be successfully conjugated with the conjugate mPEG2000-MAL.
Figure 19 shows the SDS-PAGE electropherogram of the two-site mutant BiTE-E/M-87-87 of the anti-EGFR & c-MET bispecific antibody after conjugated with mPEG2000-MAL. As can be seen from the figure, BiTE-E/M-87-87 is successfully conjugated with mPEG2000-MAL.
Figure 20 shows the molecular weight deconvolution result of the cysteine mutant Bi-TP-1-WT-87 of the anti-HER2 bispecific antibody detected by the mass spectrometry. As can be seen from the figure, the molecular weight of the mutant is 52588.4 Da.
Figure 21 shows the molecular weight deconvolution result of the cysteine mutant Bi-TP-1-WT-87 of the anti-HER2 bispecific antibody conjugated with vcMMAE detected by the mass spectrometry. As can be seen from the figure, the molecular weight of the mutant after conjugation is 53904.2 Da.
Figure 22 shows the molecular weight deconvolution result of the cysteine mutant Bi-TP-2-WT-87 of the anti-HER2 bispecific antibody detected by the mass spectrometry. As can be seen from the figure, the molecular weight of the mutant is 52002.6 Da.
Figure 23 shows the molecular weight deconvolution result of the cysteine mutant Bi-TP-2-WT-87 of the anti-HER2 bispecific antibody conjugated with vcMMAE detected by the mass spectrometry. As can be seen from the figure, the molecular weight of the mutant after conjugation is 54319.4 Da.
Figure 24 shows the molecular weight deconvolution result of the cysteine mutant Bi-TP-3-WT-87 of the anti-HER2 bispecific antibody detected by the mass spectrometry. As can be seen from the figure, the molecular weight of the mutant is 53316.1 Da.
Figure 25 shows the molecular weight deconvolution result of the cysteine mutant Bi-TP-3-WT-87 of the anti-HER2 bispecific antibody conjugated with vcMMAE detected by mass spectrometry. As can be seen from the figure, the molecular weight of the mutant after conjugation is 54632.8 Da.
Figure 26 shows the molecular weight deconvolution result of the cysteine mutant Bi-TP-4-WT-87 of the anti-HER2 bispecific antibody detected by the mass spectrometry. As can be seen from the figure, the molecular weight of the mutant is 53633.6 Da.
Figure 27 shows the molecular weight deconvolution result of the cysteine mutant Bi-TP-4-WT-87 of the anti-HER2 bispecific antibody conjugated with vcMMAE detected by the mass spectrometry. As can be seen from the figure, the molecular weight of the mutant after conjugation is 54949.5 Da.
Figure 28 shows the molecular weight deconvolution result of the cysteine mutant Tan-LH-TP-4-WT-87 of the anti-HER2 bispecific antibody detected by the mass spectrometry. As can be seen from the figure, the molecular weight of the mutant is 53533.3 Da.
Figure 29 shows the molecular weight deconvolution result of the cysteine mutant Tan-LH-TP-4-WT-87 of the anti-HER2 bispecific antibody conjugated with vcMMAE detected by the mass spectrometry. As can be seen from the figure, the molecular weight of the mutant after conjugation is 54851.2 Da.
Figure 30 shows the molecular weight deconvolution result of the cysteine mutant Tan-HL-TP-4-WT-87 of the anti-HER2 bispecific antibody detected by the mass spectrometry. As can be seen from the figure, the molecular weight of the mutant is 53533.3 Da.
Figure 31 shows the molecular weight deconvolution result of the cysteine mutant Tan-LH-TP-4-WT-87 of the anti-HER2 bispecific antibody conjugated with vcMMAE detected by the mass spectrometry. As can be seen from the figure, the molecular weight of the mutant after conjugation is 54851.4 Da.
Figure 32 shows the molecular weight deconvolution result of the cysteine mutant Tan-LH-PT-4-87-WT of the anti-HER2 bispecific antibody detected by the mass spectrometry. As can be seen from the figure, the molecular weight of the mutant is 53532.4 Da.
Figure 33 shows the molecular weight deconvolution result of the cysteine mutant Tan-LH-PT-4-87-WT of the anti-HER2 bispecific antibody conjugated with vcMMAE detected by the mass spectrometry. As can be seen from the figure, the molecular weight of the mutant after conjugation is 54849.8 Da.
Figure 34 shows the molecular weight deconvolution result of the cysteine mutant Bi-TP-4-WT-87 of the anti-HER2 bispecific antibody having a mutation at position 87 of Pertuzumab detected by the mass spectrometry. As can be seen from the figure, the molecular weight of the mutant is 53534.3 Da.
Figure 35 shows the molecular weight deconvolution result of the cysteine mutant Bi-TP-4-WT-87 of the anti-HER2 bispecific antibody having a mutation at position 87 of Pertuzumab and conjugated with MC-2MMAE detected by the mass spectrometry. As can be seen from the figure, the molecular weight of the mutant after conjugation is 56587.6 Da.
Figure 36 shows the molecular weight deconvolution result of the cysteine mutant c-MET-46C of the anti-c-MET single chain antibody fragment detected by the mass spectrometry. As can be seen from the figure, the molecular weight of the mutant is 26001.1 Da.
Figure 37 shows the molecular weight deconvolution result of the cysteine mutant c-MET-46C of the anti-c-MET single chain antibody fragment conjugated with VcMMAE detected by the mass spectrometry. As can be seen from the figure, the molecular weight of the mutant after conjugation is 27318.1 Da.
Figure 38 shows the molecular weight deconvolution result of the cysteine mutant Zalu-46C of the Zalutumumab single chain antibody fragment detected by the mass spectrometry. As can be seen from the figure, the molecular weight of the mutant is 27179.4 Da.
Figure 39 shows the molecular weight deconvolution result of the cysteine mutant Zalu-46C of the Zalutumumab single chain antibody fragment conjugated with VcMMAE detected by the mass spectrometry. As can be seen from the figure, the molecular weight of the mutant after conjugation is 28496.4 Da.

### DETAILED DESCRIPTION OF THE INVENTION

In order to make the purposes, technical solutions and advantages of the present disclosure clearer and more understandable, the present disclosure is hereinafter described in further detail in conjunction with specific examples and with reference to the drawings.

The restriction endonucleases used in the examples were purchased from Thermo Fisher Scientific (China) Inc. The reagents or materials used in the following examples can be routinely purchased in the field if the sources thereof are not explicitly mentioned.

### Definitions

"Single chain Fv", also abbreviated as "sFv" or "scFv", is an antibody fragment comprising VH and VL antibody domains linked into a single polypeptide chain. Preferably, the sFv polypeptide further comprises a polypeptide linking chain between the VH and VL domains, which enables the sFv to form an ideal structure for antigen binding.

The polypeptide linking chain is available in various forms, categorized as a flexible linking chain, a rigid linking chain and a cleavable linking chain. The most commonly used flexible linking chain is (GGGGS)n, and the other common flexible linking chains are KESGSVSSEQLAQFRSLD and EGKSSGSGSESKST, (Gly)8 or a slightly shorter (Gly)6 consisting of pure glycine. The rigid linking chains are (EAAAK)n, and (XP)n, wherein X can be specified for any amino acid, with the recommended choices being alanine (Ala), lysine (Lys) or glutamic acid (Glu). The sequences of the cleavable linking chain are LEAGCKNFFPRI↓SFTSCGSLE and the like. The linking chain used herein is the most commonly used flexible linking chain with the sequence of GGGGSGGGGSGGGGS.

In the cysteine-engineered antibodies of the present disclosure, the selected mutation sites are all located in the framework regions of the variable region of the antibodies, which is applicable to perform a cysteine mutation on said sites of any antibody, including, but not limited to, an anti-HER2 antibody, an anti-CD3 antibody, an anti-CD20 antibody, an anti-VEGFR-2 antibody, an anti-EGFR antibody, or an anti-c-Met antibody.

Wherein, HER2 (human epidermal growth factor receptor-2, ErbB2/P185), the second member of the human epidermal growth factor receptor (EGFR) family, is encoded by the proto-oncogene erbB2/Her2 localized on the human chromosome 17q21. It has a molecular weight of 185 kDa, and is a tyrosine kinase receptor membrane glycoprotein. HER2 forms a heterodimer with HER1, HER3, or HER4, leading to the activation of downstream signaling pathways such as MAPK and PI3K, and over-transmit growth signals, thereby causing the proliferation of malignant cells. HER2 proto-oncogene amplification or protein overexpression phenomenon has been found in a variety of human tumors, including 25-30% of breast cancer, 35-45% of pancreatic cancer and 90% of colorectal cancer, 16-57% of non-small cell lung cancer and 9-38% of gastric cancer, etc., which are clinically known as HER2-positive tumors, and mainly characterized by the high degree of tumor malignancy, easy progression and metastasis, insensitivity to a radiotherapy and a chemotherapy, easy recurrence, and the short survival of patients.

In 1975, Kohler and Milstein invented the hybridoma technique for the preparation of monoclonal antibody (mAb), and since then, the monoclonal antibody drugs have been rapidly developed and applied in the clinic. In recent years, the antibody drugs targeting HER2 have also become a new hotspot for the treatment of HER2-positive tumors. Trastuzumab (Herceptin) is a humanized anti-HER2 monoclonal antibody developed by Genetech Inc., which was approved by the US FDA in 1998. The combination of Trastuzumab and the chemotherapeutic drugs (Paclitaxel, etc.) has become the first-line treatment for HER2 overexpression advanced metastatic breast cancer and advanced gastric cancer at present, and the clinical effective rate in the treatment of HER2-positive metastatic breast cancer can reach 38%. The European EMEA has approved the combination of Trastuzumab and the chemotherapeutic drugs as a first-line treatment for HER2-positive progressive gastric cancer.

As used herein, the term "linker" is intended to refer to a chemical moiety comprising a covalent bond or an atomic chain that covalently attaches an antibody to a conjugated moiety (e.g., a drug moiety). In various embodiments, a linker is designated as L. A "linker" (L) is a bi-functional or multifunctional moiety that can be used to connect one or more drug moieties (D) and an antibody unit (Ab) to form an antibody-drug conjugate (ADC). Antibody-drug conjugates (ADCs) can be conveniently prepared using a linker having reactive functional groups for binding to the drug and the antibody. The thiol of cysteine of the cysteine-engineered antibody (CYSMAB) can form a bond with an electrophilic functional group of a linker reagent, a drug moiety or a drug-linker intermediate.

In one aspect, the linker has a reactive site with an electrophilic group that reacts with a nucleophilic cysteine present on the antibody. The thiol of cysteine of the antibody reacts with the electrophilic group on the linker and forms a covalent bond with the linker. Useful electrophilic groups include, but are not limited to, maleimide and haloacetamide groups. Common linkers are divided into two main categories: non-cleavable type and cleavable type. Non-cleavable linkers are mainly thioether linkers; and the cleavable linkers can be classified as the acid-cleavable, reducible and enzyme-cleavable linkers, *etc.*, among which the most commonly used cleavable linkers include Val-Cit dipeptide, etc. Mc-VC-PAB is selected as the linker herein, which is also a commonly used linker in ADC and can be purchased from MedChemExpress LLC.

The drug moiety D used in ADC herein can be a topoenzyme inhibitor (e.g., Etoposide, Teniposide, Doxorubicin, *etc.*), tubulysin A, DM1, MMAE, and the like.

In the present disclosure, tubulysin A (TubA) is a product of myxobacteria, which has the anti-angiogenic, anti-mitotic and anti-proliferative effects *in vitro.* The structure is shown below:

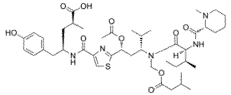

In the present disclosure, DM1, a derivative of the anti-microtubule drug Mertansine (also translated as: Maitansine or Maytansine), is a microtubule protein inhibitor that binds the microtubules at their termini and inhibits the dynamicity of the microtubules. It is an antibody-conjugatable Maytansine alkaloid that can be used to overcome the systemic toxicity associated with Mertansine and to enhance tumor specific delivery. The structure is shown below:

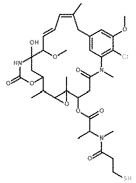

In the present disclosure, MMAE is a synthetic derivative of Dolastatin 10, which effectively inhibits mitosis by inhibiting microtubule protein polymerization. The structure is shown below:

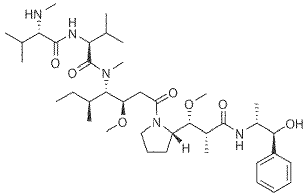

In the present disclosure, PBD, also known as pyrrolobenzodiazepine, is able to block cell division and kill cancer cells by being attached to the minor groove of DNA to form effective cytotoxic DNA interstrand crosslinks.

In the present disclosure, MMAF (Monomethylauristatin F), a potent inhibitor of tubulin polymerisation, can be used as an anti-tumor drug and is commercially available.

As used herein, the term "an active peptide", also known as a bioactive peptide, refers to a peptide compound that is beneficial to the life activities of a living organism or has a physiological effect.

"Subject", "patient", "individual" and similar terms are used interchangeably and, unless otherwise indicated, refer to the mammals such as human and non-human primate, as well as rabbit, rat, mouse, goat, pig and other mammalian species. The term does not necessarily indicate that the subject has been diagnosed with a particular disease. The term "patient" refers to a subject under medical supervision. A patient can be an individual seeking treatment, monitoring, adjustment or modification of an existing treatment regimen. A "cancer patient" or "AML patient" can refer to an individual who has been diagnosed with cancer, is currently undergoing a therapeutic regimen, or is at risk of recurrence, for example after surgery to remove a tumor. In some embodiments, the cancer patient has been diagnosed with cancer and is a candidate for therapy. The cancer patient can include the individuals who are untreated, currently undergoing treatment, or have undergone surgery, and those individuals who have discontinued treatment.

In the context of treating a cancer, a subject in need of treatment can be an individual who has a cancer or a pre-cancerous condition, already has a cancer and is at risk of recurrence, is suspected of having cancer, and is undergoing a standard cancer treatment such as radiotherapy or chemotherapy.

The terms "cancer", "tumor" and similar terms include precancerous, neoplastic and cancerous cells and can refer to solid tumors or non-solid cancers. Cancer includes both benign and malignant neoplasms (abnormal growth).

The term "cancer" can refer to leukemia, carcinoma, sarcoma, adenocarcinoma, lymphoma, solid tumors, and lymphoma, etc. Examples of different types of cancers include, but are not limited to: acute myeloid leukaemia (AML), chronic myeloid leukemia (CML), B-cell lymphoma, non-Hodgkin's lymphoma, Burkitt's lymphoma, small cell lymphoma, large cell lymphoma, monocytic leukemia, myeloid leukemia, acute lymphoblastic leukemia, multiple myeloma, lung cancer (*e*.*g*., non-small cell lung cancer or NSCLC), ovarian cancer, prostate cancer, colorectal cancer, liver cancer (*i.e.*, hepatocarcinoma), kidney cancer (*i.e.*, renal cell carcinoma), bladder cancer, breast cancer, thyroid cancer, thoracic cancer, pancreatic cancer , uterine cancer, cervical cancer, testicular cancer, anal cancer, pancreatic cancer, bile duct cancer, gastrointestinal carcinoid tumor, esophageal cancer, gallbladder cancer, appendiceal cancer, small intestine cancer, stomach (gastric) cancer, central nervous system cancer, skin cancer, choriocarcinoma, urothelial carcinoma; head and neck cancer, osteogenic sarcoma, fibrosarcoma, neuroblastoma, glioma, and melanoma.

### Example 1. Screening of the mutation sites of the anti-HER2 Trastuzumab single chain antibody fragment

The four parameters (*i.e.*, residue solvent accessibility, sidechain solvent accessibility, percent solvent accessibility and percent sidechain solvent accessibility) of the antibody were predicted using the discovery studio software based on the 4X4X model in the Protein Data Bank (PDB) herein. Considering that the reaction between nutrients and mutant cysteine during cell culture affects the subsequent conjugation, and that overly exposed sites cannot be selected, the present inventors, after comprehensive consideration, ultimately selected the sites in non-CDR regions and with all of the above four parameters ≤ 20 for mutation screening, *i.e.*, positions 12, 34, 35, 38, 44, 47, 51, 60, 61, 67, 69, 78, 79, and 114 of the heavy chain variable region; or positions 19, 21, 44, 46, 47, 48, 62, 71, 75, 78, and 87 of the light chain variable region.

### Example 2. Screening and expression of the cell lines of the cysteine mutants of the anti-HER2 Trastuzumab single chain antibody fragment

### (1) Construction of the expression vectors of the anti-HER2 Trastuzumab single chain antibody fragment and the mutants thereof

DNA encoding the antibody described herein (the corresponding wild-type sequence is obtained from USP Medicines Compendium) was used, wherein the specific nucleotide sequences of the heavy-chain variable region, the light-chain variable region, and the linking chain are set forth in SEQ ID NOs:1-3, and their amino acid sequences are set forth in SEQ ID NOs:4-6. On this basis, according to the kabat numbering, the variant HC12C of the present disclosure was obtained by substituting the amino acid at position 12 of the heavy chain with cysteine; the variant HC35C of the present disclosure was obtained by substituting the amino acid at position 35 of the heavy chain with cysteine; the variant HC61C of the present disclosure was obtained by substituting the amino acid at position 61 of the heavy chain with cysteine; the variant LC21C of the present disclosure was obtained by substituting the amino acid at position 21 of the light chain with cysteine; the variant LC47C of the present disclosure was obtained by substituting the amino acid at position 47 of the light chain with cysteine; the variant LC48C of the present disclosure was obtained by substituting the amino acid at position 48 of the light chain with cysteine; the variant LC71C of the present disclosure was obtained by substituting the amino acid at position 71 of the light chain with cysteine; the variant LC75C of the present disclosure was obtained by substituting the amino acid at position 75 of the light chain with cysteine; the variant HC34C of the present disclosure was obtained by substituting the amino acid at position 34 of the heavy chain with cysteine; the variant HC38C of the present disclosure was obtained by substituting the amino acid at position 38 of the heavy chain with cysteine; the variant HC44C of the present disclosure was obtained by substituting the amino acid at position 44 of the heavy chain with cysteine; the variant HC47C of the present disclosure was obtained by substituting the amino acid at position 47 of the heavy chain with cysteine; the variant HCS1C of the present disclosure was obtained by substituting the amino acid at position 51 of the heavy chain with cysteine; the variant HC60C of the present disclosure was obtained by substituting the amino acid at position 60 of the heavy chain with cysteine; the variant HC61C of the present disclosure was obtained by substituting the amino acid at position 61 of the heavy chain with cysteine; the variant HC67C of the present disclosure was obtained by substituting the amino acid at position 67 of the heavy chain with cysteine; the variant HC69C of the present disclosure was obtained by substituting the amino acid at position 69 of the heavy chain with cysteine; the variant HC78C of the present disclosure was obtained by substituting the amino acid at position 78 of the heavy chain with cysteine; the variant HC79C of the present disclosure was obtained by substituting the amino acid at position 79 of the heavy chain with cysteine; the variant HC114C of the present disclosure was obtained by substituting the amino acid at position 114 of the heavy chain with cysteine; the variant LC19C of the present disclosure was obtained by substituting the amino acid at position 19 of the light chain with cysteine; the variant LC44C of the present disclosure was obtained by substituting the amino acid at position 44 of the light chain with cysteine; the variant LC46C of the present disclosure was obtained by substituting the amino acid at position 46 of the light chain with cysteine; the variant LC62C of the present disclosure was obtained by substituting the amino acid at position 62 of the light chain with cysteine; the variant LC78C of the present disclosure was obtained by substituting the amino acid at position 78 of the light chain with cysteine; the variant LC87C of the present disclosure was obtained by substituting the amino acid at position 87 of the light chain with cysteine. The DNAs encoding the antibodies mentioned above were chemically synthesized (Suzhou GENEWIZ^{®} Biotechnology Co., Ltd.).

Then the genes encoding the single chain antibody fragments were double digested with HindIII and XhoI, and ligated into an eukaryotic expression vector pCGS3 (Biovector NTCC Inc.) treated with HindIII and XhoI using T4 ligase. The expression vectors of the anti-HER2 single chain antibody fragment and the mutants thereof were successfully constructed after successful ligation.

| | The coding sequence of VH | The coding sequence of VL | The sequence of the linking chain | The amino acid sequence of VH | The amino acid sequence of VL | The amino acid sequence of the linking chain |
|---|---|---|---|---|---|---|
| The WT Trastuzumab single chain antibody fragment | SEQ ID NO:1 | SEQ ID NO:2 | SEQ ID NO:3 | SEQ ID NO:4 | SEQ ID NO:5 | SEQ ID NO:6 |
| HC12C | SEQ ID NO:7 | SEQ ID NO:2 | SEQ ID NO:3 | SEQ ID NO:8 | SEQ ID NO:5 | SEQ ID NO:6 |
| HC35C | SEQ ID NO:9 | SEQ ID NO:2 | SEQ ID NO:3 | SEQ ID NO:10 | SEQ ID NO:5 | SEQ ID NO:6 |
| HC61C | SEQ ID NO:11 | SEQ ID NO:2 | SEQ ID NO:3 | SEQ ID NO:12 | SEQ ID NO:5 | SEQ ID NO:6 |
| LC21C | SEQ ID NO:1 | SEQ ID NO:13 | SEQ ID NO:3 | SEQ ID NO:4 | SEQ ID NO:14 | SEQ ID NO:6 |
| LC47C | SEQ ID NO:1 | SEQ ID NO:15 | SEQ ID NO:3 | SEQ ID NO:4 | SEQ ID NO:16 | SEQ ID NO:6 |
| LC48C | SEQ ID NO:1 | SEQ ID NO:17 | SEQ ID NO:3 | SEQ ID NO:4 | SEQ ID NO:18 | SEQ ID NO:6 |
| LC71C | SEQ ID NO:1 | SEQ ID NO:19 | SEQ ID NO:3 | SEQ ID NO:4 | SEQ ID NO:20 | SEQ ID NO:6 |
| LC75C | SEQ ID NO:1 | SEQ ID NO:21 | SEQ ID NO:3 | SEQ ID NO:4 | SEQ ID NO:22 | SEQ ID NO:6 |
| HC34C | SEQ ID NO:23 | SEQ ID NO:2 | SEQ ID NO:3 | SEQ ID NO:24 | SEQ ID NO:5 | SEQ ID NO:6 |
| HC38C | SEQ ID NO:25 | SEQ ID NO:2 | SEQ ID NO:3 | SEQ ID NO:26 | SEQ ID NO:5 | SEQ ID NO:6 |
| HC44C | SEQ ID NO:27 | SEQ ID NO:2 | SEQ ID NO:3 | SEQ ID NO:28 | SEQ ID NO:5 | SEQ ID NO:6 |
| HC47C | SEQ ID NO:29 | SEQ ID NO:2 | SEQ ID NO:3 | SEQ ID NO:30 | SEQ ID NO:5 | SEQ ID NO:6 |
| HC51C | SEQ ID NO:31 | SEQ ID NO:2 | SEQ ID NO:3 | SEQ ID NO:32 | SEQ ID NO:5 | SEQ ID NO:6 |
| HC60C | SEQ ID NO:33 | SEQ ID NO:2 | SEQ ID NO:3 | SEQ ID NO:34 | SEQ ID NO:5 | SEQ ID NO:6 |
| HC67C | SEQ ID NO:35 | SEQ ID NO:2 | SEQ ID NO:3 | SEQ ID NO:36 | SEQ ID NO:5 | SEQ ID NO:6 |
| HC69C | SEQ ID NO:37 | SEQ ID NO:2 | SEQ ID NO:3 | SEQ ID NO:38 | SEQ ID NO:5 | SEQ ID NO:6 |
| HC78C | SEQ ID NO:39 | SEQ ID NO:2 | SEQ ID NO:3 | SEQ ID NO:40 | SEQ ID NO:5 | SEQ ID NO:6 |
| HC79C | SEQ ID NO:41 | SEQ ID NO:2 | SEQ ID NO:3 | SEQ ID NO:42 | SEQ ID NO:5 | SEQ ID NO:6 |
| HC114C | SEQ ID NO:43 | SEQ ID NO:2 | SEQ ID NO:3 | SEQ ID NO:44 | SEQ ID NO:5 | SEQ ID NO:6 |
| LC19C | SEQ ID NO:1 | SEQ ID NO:45 | SEQ ID NO:3 | SEQ ID NO:4 | SEQ ID NO:46 | SEQ ID NO:6 |
| LC44C | SEQ ID NO:1 | SEQ ID NO:47 | SEQ ID NO:3 | SEQ ID NO:4 | SEQ ID NO:48 | SEQ ID NO:6 |
| LC46C | SEQ ID NO:1 | SEQ ID NO:49 | SEQ ID NO:3 | SEQ ID NO:4 | SEQ ID NO:50 | SEQ ID NO:6 |
| LC62C | SEQ ID NO:1 | SEQ ID NO:51 | SEQ ID NO:3 | SEQ ID NO:4 | SEQ ID NO:52 | SEQ ID NO:6 |
| LC78C | SEQ ID NO:1 | SEQ ID NO:53 | SEQ ID NO:3 | SEQ ID NO:4 | SEQ ID NO:54 | SEQ ID NO:6 |
| LC87C | SEQ ID NO:1 | SEQ ID NO:55 | SEQ ID NO:3 | SEQ ID NO:4 | SEQ ID NO:56 | SEQ ID NO:6 |

### (2) Screening and purification of the expression vectors of the anti-HER2 Trastuzumab single chain antibody fragment and the mutants thereof

The successfully constructed expression vectors were transferred into DH5α competent *E*. *coli* strains (purchased from Beijing Zoman Biotechnology Co., Ltd.). The transformed DH5α strains were verified by double digestion with two enzymes of HindIII and XhoI and sequencing to select positive clones. The plasmids were extracted by lysing the resulting *E*. *coli* using a plasmid extraction kit (purchased from CoWin) to obtain purified expression vectors.

### (3) Expression of the anti-HER2 Trastuzumab single chain antibody fragment and the mutants thereof in CHO cells

The purified expression vectors were transfected into CHO-K1 (purchased from ATCC) cells by electroporation. The cells were seeded to 96-well plates, and grown for 15 days to pick out the monoclones. The antibody yields of the cells were determined by western blot. Cells with the top 20% of the yields were selected and transferred to 24-well plates. After 7 days of cell growth, the antibody yields of the cells were detected. Cells with the top 5 to 6 of the yields were selected and transferred to the shake flasks for culture. After the serum-free culture of the cells was stabilized, the cells were harvested after 7 days of continuous culture to achieve the expression of anti-HER2 single chain antibody fragment and mutants thereof in the CHO-K1 cells. The antibodies were purified, and the subsequent experiments were performed on them.

### Example 3. Purification using Ni affinity chromatography column

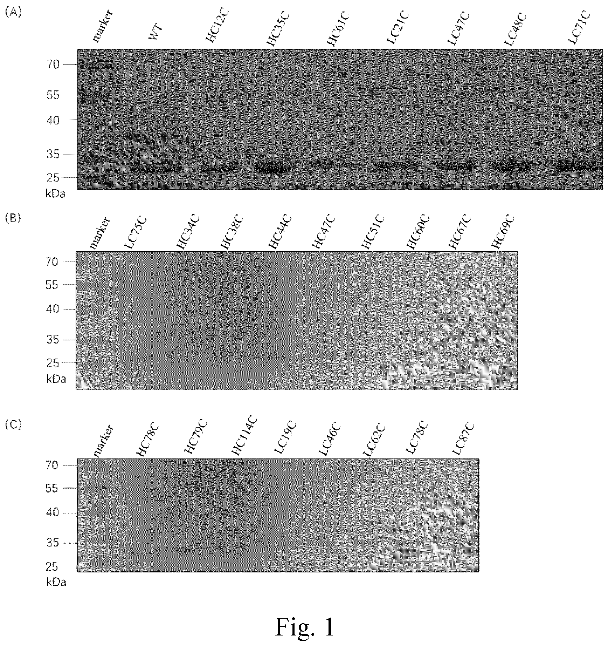

The cells and culture medium were transferred into a 50 mL centrifuge tube, placed in a high speed refrigerated centrifuge, and centrifuged at 8000r for 15 min. The supernatant was remained, and the cell debris was discarded. The culture medium was diluted with an equilibrium solution of 3 times volume of the culture medium, followed by filtration with a 0.22 µm microporous filter membrane once. The filtrate was collected for later use. The pump, the sample injector and the purification column were washed with the ultrapure water and PBS of 10 times of column volume, and waited for sampling. The sample was loaded. The column was eluted with an eluent containing 250 mM imidazole and the purified antibody protein solution was picked up into an EP tube and stored at 2-8°C. The results of SDS-PAGE purity detection are shown in Figure 1. As can be seen from the electrophoretograms, the antibody bands are single and the purity meets the needs of subsequent experiments.

### Example 4. Detection of the proportion of the antibody free sulfhydryl group

Ellman's method (Thermo Fisher Scientific (China) Inc.) is used in the present disclosure to detect the content of the free sulfhydryl groups in antibody products so as to select cell lines suitable for further conjugation. A 0.1M Na₂HPO₄ solution, a DTNB (10 mM) solution, and a cysteine standard solution with a final concentration of 0.0059 M~0.375 M were formulated. Antibody samples to be detected were also prepared at a concentration of 5 mg/mL. 250µL 0.1M Na₂HPO₄ and 5µL DTNB solutions were added into 1.5mL EP tube containing the cysteine standard solution and the sample to be detected, vortexed and shaken to mix well and incubated for 5 min at room temperature. The OD value was detected at the wavelength of 412 nm. A standard curve was established based on the OD values, and the sulfhydryl content of the sample was calculated.

**Table 1. Detection of the content of the free sulfhydryl groups in the antibody products using Ellman's method**

| **Sample** | **the free sulfhydryl group%** |
|---|---|
| WT | 3.28% |
| HC12C | 93.22% |
| HC35C | 87.70% |
| HC61C | 96.51% |
| LC21C | 24.35% |
| LC47C | 36.38% |
| LC48C | 19.07% |
| LC71C | 46.17% |
| LC75C | 18.41% |
| HC34C | 19.33% |
| HC38C | 29.63% |
| HC44C | 47.24% |
| HC47C | 34.51% |
| HC51C | 85.61% |
| HC60C | 30.67% |
| HC67C | 23.82% |
| HC69C | 57.11% |
| HC78C | 72.24% |
| HC79C | 35.28% |
| HC114C | 30.24% |
| LC19C | 11.56% |
| LC46C | 96.51% |
| LC62C | 32.67% |
| LC78C | 36.48% |
| LC87C | 97.32% |

As can be seen in Table 1, the wild type essentially does not contain the free sulfhydryl groups, and the cysteine-engineered mutants all have the free sulfhydryl groups available for conjugation.

### Example 5. Determination of the antibody-antigen affinity

The affinity of the wild-type antibody and the cysteine-engineered antibody mutants to the antigen HER-FC was determined by bio-layer interferometry (BLI) using Pro A probe (ForteBio).

HER-FC (Beijing Sino Biological Inc.) was dissolved in PBS at a working concentration of 10 µg/mL. The antibodies were dissolved in PBS2, and diluted gradiently to the concentrations of 18.75 nM, 37.5 nM, 75 nM, 150 nM, and 300 nM, respectively. All of the working volumes were 200 µL.

The data plots were fitted by OCTET system and the data processing software. The intermolecular forces between the antibodies and HER2 were calculated using the software, and expressed as KD values. The results are shown in Table 2.

**Table 2. Affinity of the wild-type antibodies and the mutants to the antigens**

| **Sample** | **The KD of the antibody to the antigen (10⁻⁹)** |
|---|---|
| WT | 8.78 |
| HC12C | 6.21 |
| HC35C | 17.1 |
| HC61C | 7.26 |
| LC21C | 6.28 |
| LC47C | 5.96 |
| LC48C | 9.28 |
| LC71C | 8.47 |
| LC75C | 6.51 |
| LC46C | 5.81 |
| LC87C | 2.02 |

As can be seen from the data in the table, there is almost no difference between the affinity of the wild-type antibody to the antigen and the affinity of the mutants to the antigen. Therefore, the mutations at selected sites do not change the effect of the antibody.

### Example 6. Antibody-antigen binding assay

The binding ability of the wild-type antibody and the cysteine-engineered antibody mutants to the antigen HER-Fc (Beijing Sino Biological Inc.) was determined using the enzyme-linked immunosorbent assay (ELISA). The results are shown in Table 3.

**Table 3. The binding ability of the wild-type antibody and the mutants to the antigen**

| **Sample** | **The EC₅₀ value (10⁻⁹)** |
|---|---|
| WT | 0.48 |
| HC12C | 0.91 |
| HC35C | 5.02 |
| HC61C | 0.61 |
| LC21C | 0.76 |
| LC47C | 0.48 |
| LC48C | 0.53 |
| LC71C | 0.81 |
| LC75C | 0.74 |
| LC46C | 0.65 |
| LC87C | 0.64 |

As can be seen from the data in the table, there is almost no difference between the binding ability of the wild-type antibody to the antigen and that of the mutants to the antigen. Therefore, the mutations at selected sites do not change the effect of the antibody.

### Example 7. Screening of the cell lines of the two-site cysteine mutants of the anti-HER2 Trastuzumab single chain antibody fragment

On the basis of Examples 1-6, the present disclosure also carried out a two-site cysteine mutation on the anti-HER2 Trastuzumab single chain antibody fragment as shown in the table below.

**Table 4. Two-site mutations of the antibody and the names thereof**

| The mutation sites | The abbreviation for the name |
|---|---|
| The amino acid at position 12 of the heavy chain changes from Val to Cys, and the amino acid at position 35 of the heavy chain changes from His to Cys. | 12C-35C |
| The amino acid at position 12 of the heavy chain changes from Val to Cys, and the amino acid at position 61 of the heavy chain changes from Ala to Cy. | 12C-61C |
| The amino acid at position 35 of the heavy chain changes from His to Cys, and the amino acid at position 61 of the heavy chain changes from Ala to Cys. | 35C-61C |
| The amino acid at position 51 of the heavy chain changes from IIe to Cys, and the amino acid at position 61 of the heavy chain changes from Ala to Cys. | 51C-61C |
| The amino acid at position 51 of the heavy chain changes from IIe to Cys, and the amino acid at position 46 of the light chain changes from Leu to Cys. | 51C-46C |
| The amino acid at position 61 of the heavy chain changes from Ala to Cys, and the amino acid at position 46 of the light chain changes from Leu to Cys. | 61C-46C |
| The amino acid at position 61 of the heavy chain changes from Ala to Cys, and the amino acid at position 87 of the light chain changes from Tyr to Cys. | 61C-87C |
| The amino acid at position 21 of the light chain changes from Ile to Cys, and the amino acid at position 47 of the light chain changes from Leu to Cys. | 21C-47C |
| The amino acid at position 21 of the light chain changes from Ile to Cys, and the amino acid at position 48 of the light chain changes from Ile to Cys. | 21C-48C |
| The amino acid at position 21 of the light chain changes from Ile to Cys, and the amino acid at position 75 of the light chain changes from Ile to Cys. | 21C-75C |
| The amino acid at position 46 of the light chain changes from Leu to Cys, and the amino acid at position 87 of the light chain changes from Tyr to Cys. | 46C-87C |

### (1) Construction of the expression vectors of the anti-HER2 Trastuzumab single chain antibody fragment and the two-site mutants thereof

DNA encoding the antibody described herein (the corresponding wild-type sequence is obtained from USP Medicines Compendium) was used, wherein the specific nucleotide sequences of the heavy-chain variable region, the light-chain variable region, and the linking chain are set forth in SEQ ID NOs:1-3, and their amino acid sequences are set forth in SEQ ID NOs:4-6. On this basis, according to the kabat numbering, the variant 12C-35C of the present disclosure was obtained by substituting the amino acids at positions 12 and 15 of the heavy chain with cysteine; the variant 12C-61C of the present disclosure was obtained by substituting the amino acids at positions 12 and 61 of the heavy chain with cysteine; the variant 35C-61C of the present disclosure was obtained by substituting the amino acids at positions 35 and 61 of the heavy chain with cysteine; the variant 51C-61C of the present disclosure was obtained by substituting the amino acids at positions 51 and 61 of the heavy chain with cysteine; the variant 51C-46C of the present disclosure was obtained by substituting the amino acid at position 51 of the heavy chain and the amino acid at position 46 of the light chain with cysteine; the variant 61C-46C of the present disclosure was obtained by substituting the amino acid at position 61 of the heavy chain and the amino acid at position 46 of the light chain with cysteine; the variant 61C-87C of the present disclosure was obtained by substituting the amino acid at position 61 of the heavy chain and the amino acid at position 87 of the light chain with cysteine; the variant 21C-47C of the present disclosure was obtained by substituting the amino acid at positions 21 and 47 of the light chain with cysteine; the variant 21C-48C of the present disclosure was obtained by substituting the amino acid at positions 21 and 48 of the light chain with cysteine; the variant 21C-75C of the present disclosure was obtained by substituting the amino acid at positions 21 and 75 of the light chain with cysteine; the variant 46C-87C of the present disclosure was obtained by substituting the amino acid at positions 46 and 87 of the light chain with cysteine. The DNAs encoding the antibodies mentioned above were chemically synthesized (Suzhou GENEWIZ^{®} Biotechnology Co., Ltd.). The sequence listing is shown below.

Then the genes encoding the single chain antibody fragments were double digested with HindIII and XhoI, and ligated into an eukaryotic expression vector pCGS3 (Biovector NTCC Inc.) treated with HindIII and XhoI using T4 ligase. The expression vectors of the anti-HER2 single chain antibody fragment and the two-site mutants thereof were successfully constructed after successful ligation.

| | The coding sequence of VH | The coding sequence of VL | The sequence of the linking chain | The amino acid sequence of VH | The amino acid sequence of VL | The amino acid sequence of the linking chain |
|---|---|---|---|---|---|---|
| 12C-35C | SEQ ID NO:57 | SEQ ID NO:2 | SEQ ID NO:3 | SEQ ID NO:58 | SEQ ID NO:5 | SEQ ID NO:6 |
| 12C-61C | SEQ ID NO:59 | SEQ ID NO:2 | SEQ ID NO:3 | SEQ ID NO:60 | SEQ ID NO:5 | SEQ ID NO:6 |
| 35C-61C | SEQ ID NO:61 | SEQ ID NO:2 | SEQ ID NO:3 | SEQ ID NO:62 | SEQ ID NO:5 | SEQ ID NO:6 |
| 51C-61C | SEQ ID NO:63 | SEQ ID NO:2 | SEQ ID NO:3 | SEQ ID NO:64 | SEQ ID NO:5 | SEQ ID NO:6 |
| 51C-46C | SEQ ID NO:31 | SEQ ID NO:49 | SEQ ID NO:3 | SEQ ID NO:32 | SEQ ID NO:50 | SEQ ID NO:6 |
| 61C-46C | SEQ ID NO:11 | SEQ ID NO:49 | SEQ ID NO:3 | SEQ ID NO:12 | SEQ ID NO:50 | SEQ ID NO:6 |
| 61C-87C | SEQ ID NO:11 | SEQ ID NO:55 | SEQ ID NO:3 | SEQ ID NO:12 | SEQ ID NO:56 | SEQ ID NO:6 |
| 21C-47C | SEQ ID NO:1 | SEQ ID NO:65 | SEQ ID NO:3 | SEQ ID NO:4 | SEQ ID NO:66 | SEQ ID NO:6 |
| 21C-48C | SEQ ID NO:1 | SEQ ID NO:67 | SEQ ID NO:3 | SEQ ID NO:4 | SEQ ID NO:68 | SEQ ID NO:6 |
| 21C-75C | SEQ ID NO:1 | SEQ ID NO:69 | SEQ ID NO:3 | SEQ ID NO: 4 | SEQ ID NO:70 | SEQ ID NO:6 |
| 46C-87C | SEQ ID NO:1 | SEQ ID NO:71 | SEQ ID NO:3 | SEQ ID NO:4 | SEQ ID NO:72 | SEQ ID NO:6 |

### (2) Screening and purification of the expression vectors of the anti-HER2 Trastuzumab single chain antibody fragment and the two-site mutants thereof

The successfully constructed expression vectors were transferred into DH5α competent E. *coli* strains. The transformed DH5α strains were verified by double digestion with two enzymes of HindIII and XhoI and sequencing to select positive clones. The plasmids were extracted by lysing the resulting *E*. *coli* using a plasmid extraction kit (purchased from CoWin) to obtain purified expression vectors.

### (3) Expression of the anti-HER2 Trastuzumab single chain antibody fragment and the two-site mutants thereof in CHO cells

The purified expression vector was transfected into CHO-K1 (purchased from ATCC) cells by electroporation. The cells were seeded to 96-well plates, and grown for 15 days to pick out the monoclones. The antibody yields of the cells were determined by western blot. Cells with the top 20% of the yields were selected and transferred to 24-well plates. After 7 days of cell growth, the antibody yields of the cells were detected. Cells with the top 5 to 6 of the yields were selected and transferred to the shake flasks for culture. After the serum-free culture of the cells was stabilized, the cells were harvested after 7 days of continuous culture to achieve the expression of anti-HER2 single chain antibody fragments in the CHO-K1 cells. The antibodies were purified, and the subsequent experiments were performed on them.

The two-site mutants of the anti-HER2 Trastuzumab single chain antibody fragment also have similar technical effects to those of the single-site mutants described in Examples 1-6. Their conjugating effects can be found in the experimental results of Example 11 "Conjugation of the antibody with vcMMAE and detection thereof" and Example 13 "Detection of cell proliferation inhibition".

### Example 8. Screening of the cell lines of the cysteine mutants of the other single chain antibody fragments

Examples 1-7 have verified each mutation site using the anti-HER2 Trastuzumab single chain antibody fragment as an example. Mutations were also performed on the corresponding sites of the other antibodies in the present disclosure. It was verified that the above mutated sites have universality on various antibodies. Specifically, in the present disclosure, the cysteine mutations were performed on the amino acids at positions 46 and 87 of the light chain of the Pertuzumab single chain antibody fragment, with the mutants thereof naming Per-46C and Per-87C, respectively; a cysteine mutation was performed on the amino acid at position 46 of the light chain of the Rituximab single chain antibody fragment, with the mutant thereof naming Rit-46C; a cysteine mutation was performed on the amino acid at position 46 of the light chain of the Muromonab single chain antibody fragment, with the mutant thereof naming Mur-46C; and a cysteine mutation was performed on the amino acid at position 87 of the light chain of the Ramucirumab single chain antibody fragment, with the mutant thereof naming Ram-87C. The sequence listing is shown below.

| | The coding sequence of VH | The coding sequence of VL | The sequence of the linking chain | The amino acid sequence of VH | The amino acid sequence of VL | The amino acid sequence of the linking chain |
|---|---|---|---|---|---|---|
| Per-46C | SEQ ID NO:73 | SEQ ID NO:74 | SEQ ID NO:3 | SEQ ID NO:75 | SEQ ID NO:76 | SEQ ID NO:6 |
| Per-87C | SEQ ID NO:73 | SEQ ID NO:77 | SEQ ID NO:3 | SEQ ID NO:75 | SEQ ID NO:78 | SEQ ID NO:6 |
| Rit-46C | SEQ ID NO:79 | SEQ ID NO:80 | SEQ ID NO:3 | SEQ ID NO:81 | SEQ ID NO:82 | SEQ ID NO:6 |
| Mur-46C | SEQ ID NO:83 | SEQ ID NO:84 | SEQ ID NO:3 | SEQ ID NO:85 | SEQ ID NO:86 | SEQ ID NO:6 |
| Ram-87C | SEQ ID NO:87 | SEQ ID NO:88 | SEQ ID NO:3 | SEQ ID NO:89 | SEQ ID NO:90 | SEQ ID NO:6 |

Mutants of the corresponding sites of the Pertuzumab single chain antibody fragment, the Rituximab single chain antibody fragment, the Muromonab single chain antibody fragment, and the Ramucirumab single chain antibody fragment also have similar effects to that of the anti-HER2 Trastuzumab single chain antibody fragment. Their conjugating effects can be found in the experimental results of "Example 12. Conjugation of the other antibodies in the form of the single chain antibody fragment with mPEG2000-MAL and detection thereof". Therefore, the above mutation sites screened by the present disclosure have universality on various antibodies.

### Example 9. Screening of the cell lines of the two-site cysteine mutants of the anti-HER2 single chain bispecific antibody

A bispecific antibody in the form of BiTE was constructed based on the anti-HER2 Trastuzumab single chain antibody fragment and the anti-HER2 Pertuzumab single chain antibody fragment, and designated as BiTE-WT. The cysteine mutations were performed on the amino acid at position 46 or 87 of the light chains of the anti-HER2 Trastuzumab single chain antibody fragment and the anti-HER2 Pertuzumab single chain antibody fragment, respectively, to construct the cysteine mutant bispecific antibody in the form of BiTE. For the cysteine mutant bispecific antibodies in the form of BiTE, the names are written with the VL mutation site in Trastuzumab antibody in front of the VL mutation site in Pertuzumab antibody. They were designated as BiTE-46-46, BiTE-46-87, BiTE-87-46, and BiTE-87-87, respectively.

BiTE was linked in the form of Herceptin VL- GGGGSGGGGSGGGGS- Herceptin VH-GGGGS- Perjeta VH- GGGGSGGGGSGGGGS- Perjeta VL, with the sequences listed below.

| Name | The coding sequenc e of Her-VH | The coding sequenc e of Her-VL | The coding sequence of Her-in-chain linker | The amino acid sequence of Her-VH | The amino acid sequence of Her-VL | The amino acid sequence of Her-in-chain linker | The coding sequenc e of Per-VH | The coding sequence of Per-VL | The coding sequence of Per-in-chain linker | The amino acid sequenc e of Per-VH | The amino acid sequenc e of Per-VL | The amino acid sequence of Per-in-chain linker | The coding sequence of Her-Per interchain linker | The amino acid sequence of Her-Per interchain linker |
|---|---|---|---|---|---|---|---|---|---|---|---|---|---|---|
| BiTE-WT | SEQ ID NO:1 | SEQ ID NO:2 | SEQ ID NO:3 | SEQ ID NO:4 | SEQ ID NO:5 | SEQ ID NO:6 | SEQ ID NO:73 | SEQ ID NO:91 | SEQ ID NO:3 | SEQ ID NO:75 | SEQ ID NO:92 | SEQ ID NO:6 | SEQ ID NO:93 | SEQ ID NO:94 |
| BiTE-46-46 | SEQ ID NO:1 | SEQ ID NO:49 | SEQ ID NO:3 | SEQ ID NO:4 | SEQ ID NO:50 | SEQ ID NO:6 | SEQ ID NO:73 | SEQ ID NO:74 | SEQ ID NO:3 | SEQ ID NO:75 | SEQ ID NO:76 | SEQ ID NO:6 | SEQ ID NO:93 | SEQ ID NO:94 |
| BiTE-46-87 | SEQ ID NO:1 | SEQ ID NO:49 | SEQ ID NO:3 | SEQ ID NO:4 | SEQ ID NO:50 | SEQ ID NO:6 | SEQ ID NO:73 | SEQ ID NO:77 | SEQ ID NO:3 | SEQ ID NO:75 | SEQ ID NO:78 | SEQ ID NO:6 | SEQ ID NO:93 | SEQ ID NO:94 |
| BiTE-87-46 | SEQ ID NO:1 | SEQ ID NO:55 | SEQ ID NO:3 | SEQ ID NO:4 | SEQ ID NO:56 | SEQ ID NO:6 | SEQ ID NO:73 | SEQ ID NO:74 | SEQ ID NO:3 | SEQ ID NO:75 | SEQ ID NO:76 | SEQ ID NO:6 | SEQ ID NO:93 | SEQ ID NO:94 |
| BiTE-87-87 | SEQ ID NO:1 | SEQ ID NO:55 | SEQ ID NO:3 | SEQ ID NO:4 | SEQ ID NO:56 | SEQ ID NO:6 | SEQ ID NO:73 | SEQ ID NO:77 | SEQ ID NO:3 | SEQ ID NO:75 | SEQ ID NO:78 | SEQ ID NO:6 | SEQ ID NO:93 | SEQ ID NO:94 |

The two-site mutants of the anti-HER2 single chain bispecific antibody also have similar technical effects to those of the single-site mutants of the monospecific antibody described in Examples 1-6. Their conjugating effects can be found in the experimental results of Example 11 "Conjugation of the antibody with vcMMAE and detection thereof" and Example 13 "Detection of cell proliferation inhibition".

### Example 10. Conjugation of the anti-HER2 Trastuzumab single chain antibody fragments with mPEG2000-MAL and detection thereof

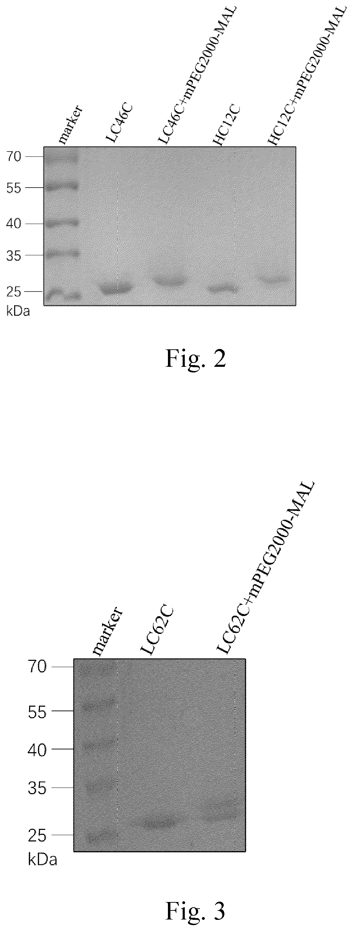

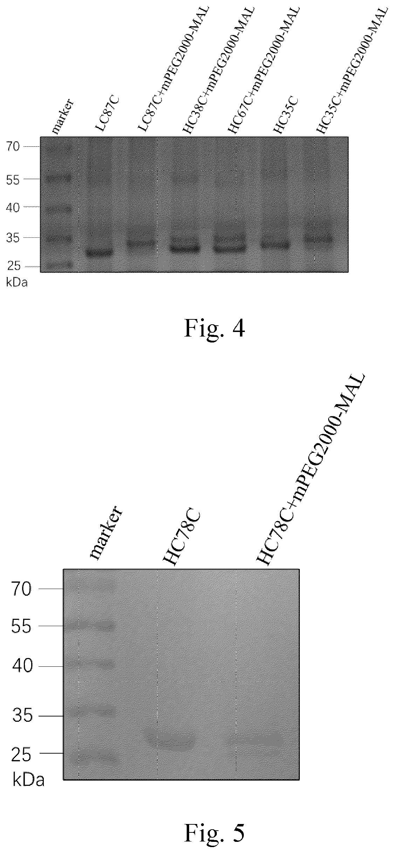

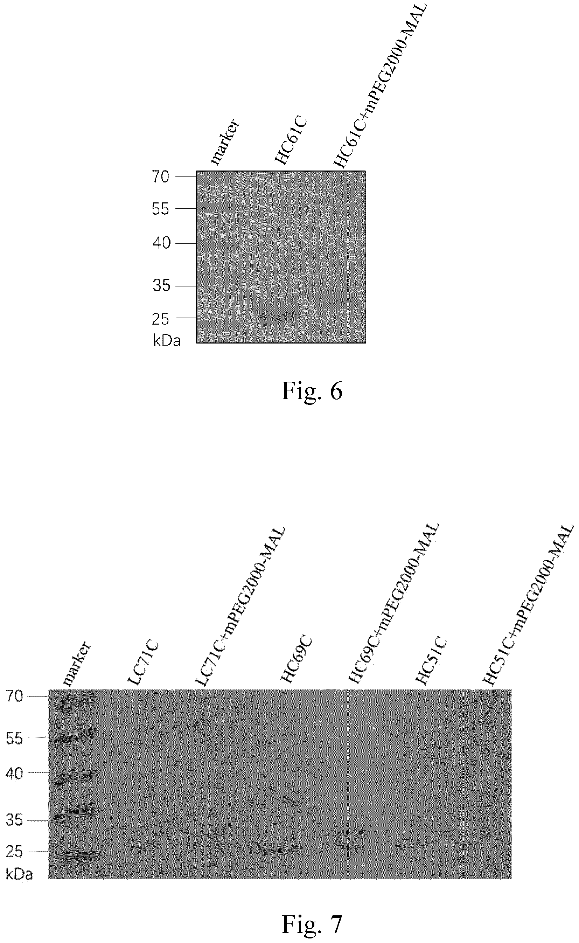

In this example, the purified single-site mutants of anti-HER2 Trastuzumab single chain antibody fragment obtained in Example 3 were used as examples, and mPEG2000-MAL was used as a conjugated moiety to carry out the experiments. Wherein, the conjugated reaction conditions of the antibody and the conjugated moiety were: the antibody: the conjugated moiety (mPEG2000-MAL)=1:30 (molar ratio), the reaction temperature of 37 °C, the reaction time of 3h, and the reaction buffer being PBS (pH 7.2). The SDS-PAGE electropherograms of the wild-type or mutants of single chain antibody fragments conjugated with mPEG2000-MAL (purchased from Shanghai STANDARDS Biotechnology Co., Ltd., the product identification number: ZZP-MPEG-MAL-2K-01) are shown in Figures 2-8, respectively.

The conjugation of the mutants of single chain antibody fragments with mPEG2000-MAL was detected by SDS-PAGE experiment herein. The protein bands shifted upward after conjugation, indicating an increase in the molecular weight. The results show that each mutant can be successfully conjugated with mPEG2000-MAL. Based on the results of the conjugation of the antibodies with mPEG2000-MAL, it is known that the mutants of single chain antibody fragments herein have the reactive sulfhydryl groups, which can react with the drug linkers having sulfhydryl-reactive functional groups (*e*.*g*., maleimide functional groups). Thus, it can be reasonably expected that they can also realize the linkage between the antibodies and the drug molecules.

### Example 11. Conjugation of the antibody with vcMMAE and detection thereof

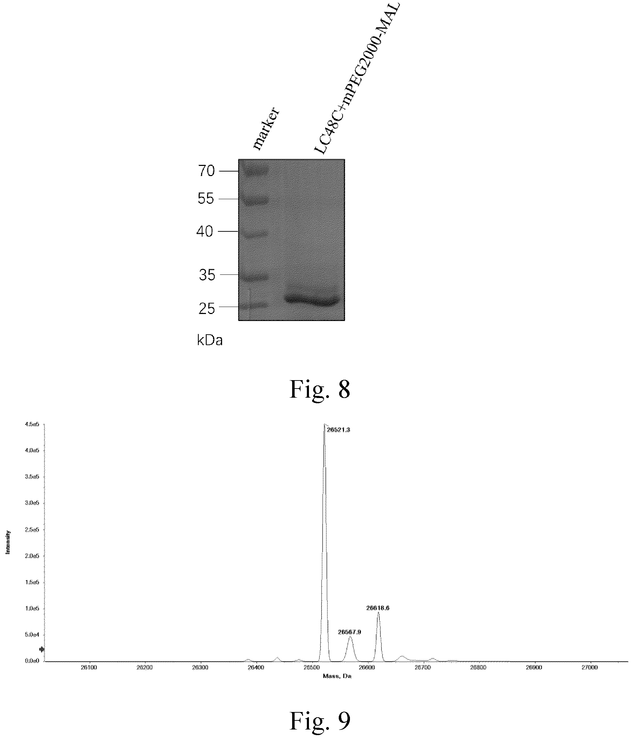

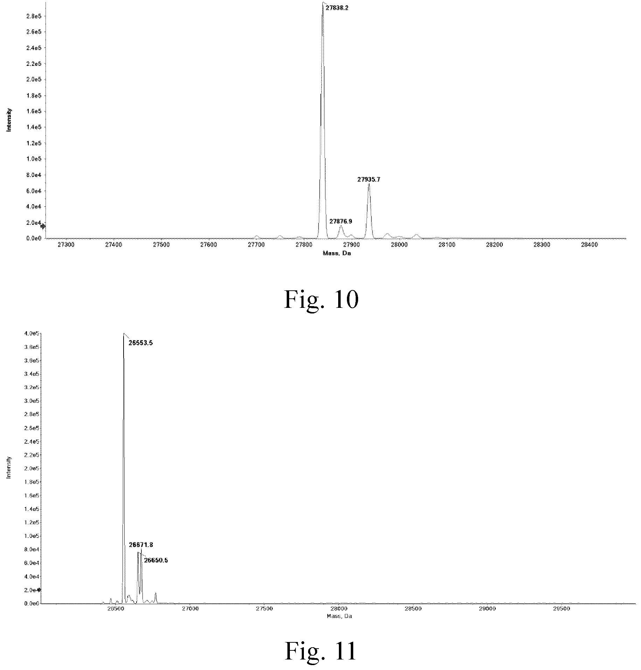

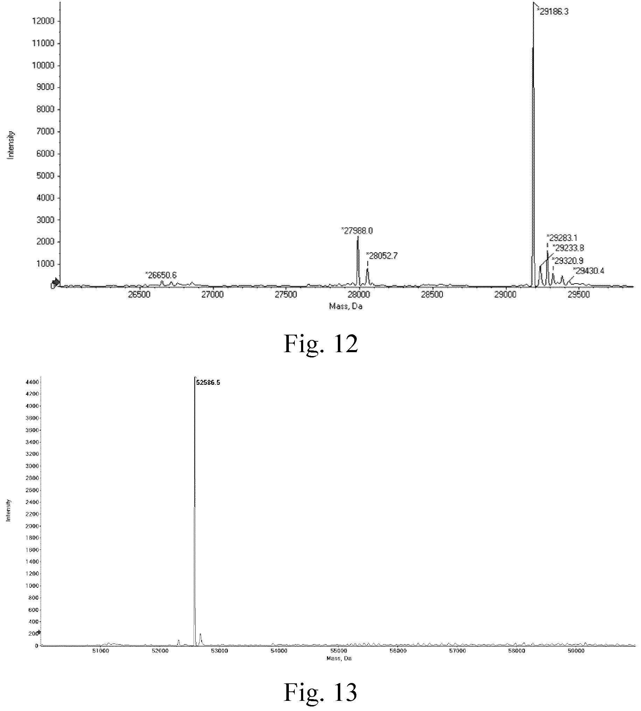

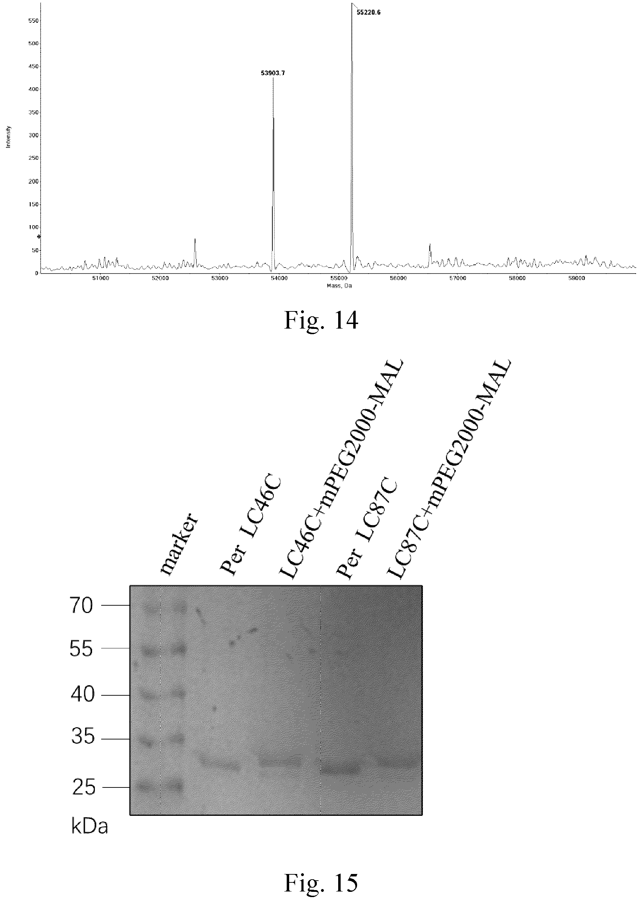

In this example, the purified single-site mutant of the anti-HER2 Trastuzumab single chain antibody fragment (LC87) obtained in Example 3, the purified two-site mutant of the anti-HER2 Trastuzumab single chain antibody fragment (HC61C-LC87C) obtained in Example 7, and the purified two-site mutant bispecific antibody (BiTE-87-87) obtained in Example 9, were selected as examples, and the drug molecule vcMMAE (purchased from Nanjing Adooq Bioscience Co., Ltd., the product identification number: A14362) was used as a conjugated moiety to carry out the experiments. Wherein, the conjugated reaction conditions of the antibody and the conjugated moiety were: the antibody: the conjugated moiety (vcMMAE)=1:5 (molar ratio), the reaction temperature of 37°C, the reaction time of 3h, and the reaction buffer being PBS (pH 7.2). Typical mass spectra of the single-site mutant of single chain antibody fragment before and after conjugated with vcMMAE are shown in Figures 9 and 10, wherein Figure 9 is a result image showing the molecular weight of the single chain antibody fragment before conjugation, with the molecular weight of the single chain antibody fragment being 26521.3 Da; and Figure 10 is a result image showing the molecular weight of the antibody after conjugated with vcMMAE, with the molecular weight of the antibody after conjugated with vcMMAE being 27838.2 Da, and the calculated difference was 1316.9Da, which is the molecular weight of vcMMAE, proving that each mutant of single chain antibody fragment was successfully conjugated with one molecule of vcMMAE. Typical mass spectra of the two-site mutant before and after the conjugation are shown in Figures 11 and 12, wherein Figure 11 is the result image showing the molecular weight of the single chain antibody fragment before conjugation, with the molecular weight of the single chain antibody fragment being 26,553.5Da; and Figure 12 is the result image showing the molecular weight of the antibody after conjugated with vcMMAE, with the molecular weights of the antibody after conjugated with vcMMAE being 27,988.0Da and 29,186.3Da, and the calculated differences are the molecular weights of 1 and 2 molecules of vcMMAE, respectively, proving that each two-site mutant of single chain antibody fragment was successfully conjugated with more than 1.7 vcMMAE. Typical mass spectra of the two-site mutant of bispecific antibody before and after the conjugation are shown in Figures 13 and 14, wherein Figure 13 is the result image showing the molecular weight of the two-site mutant of bispecific antibody before conjugation, with the molecular weight of the single chain antibody fragment being 52588.5Da; and Figure 14 is the result image showing the molecular weight of the antibody after conjugated with vcMMAE, with the molecular weights of the antibody after conjugated with vcMMAE being 53903.7Da and 55220.6Da, and the calculated differences are the molecular weights of 1 and 2 molecules of vcMMAE, respectively, proving that each two-site mutant of bispecific antibody was successfully conjugated with more than 1.5 vcMMAE.

### Example 12. Conjugation of the other antibodies in the form of the single chain antibody fragment with mPEG2000-MAL and detection thereof

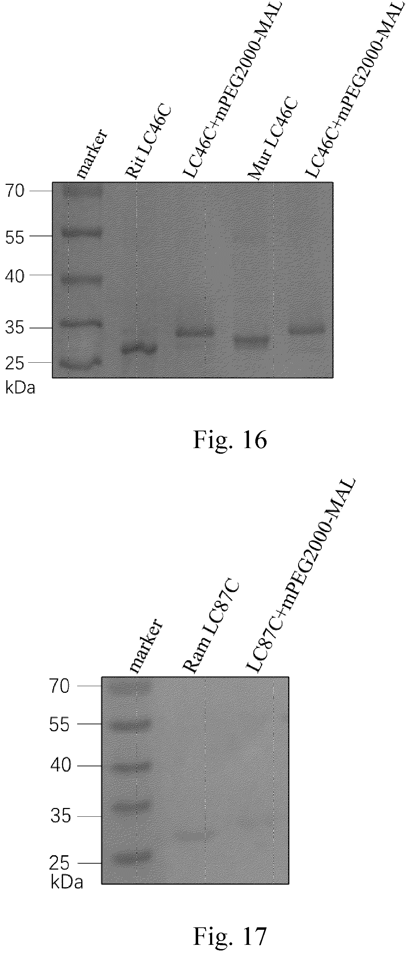

In this Example, the mutants of the Pertuzumab single chain antibody fragment, the Rituximab single chain antibody fragment, the Muromonab single chain antibody fragment, and the Ramucirumab single chain antibody fragment obtained in Example 8 were used as examples, and mPEG2000-MAL (purchased from Shanghai STANDARDS Biotechnology Co., Ltd., the product identification number: ZZP-MPEG-MAL-2K-01) was used as a conjugated moiety to carry out the experiments. Wherein, the cysteine mutations were performed on the amino acids at positions 46 and 87 of the light chain of Pertuzumab; the cysteine mutation was performed on the amino acid at position 46 of the light chain of Rituximab; the cysteine mutation was performed on the amino acid at position 46 of the light chain of Muromonab; and the cysteine mutation was performed on the amino acid at position 87 of the light chain of Ramucirumab. The conjugated reaction conditions of the obtained antibody mutants and the conjugated moiety were: the antibody: the conjugated moiety (mPEG2000-MAL)=1:30 (molar ratio), the reaction temperature of 37°C, the reaction time of 3h, and the reaction buffer being PBS (pH 7.2). Wherein, the SDS-PAGE electropherograms of the mutants of single chain antibody fragments conjugated with mPEG2000-MAL are shown in Figures 15-17. The conjugation of the mutants of single chain antibody fragments with mPEG2000-MAL was detected using the SDS-PAGE experiments. The protein bands shifted upward after conjugation, indicating an increase of the molecular weight. The results show that the mutants of single chain antibody fragments of four antibodies can be successfully conjugated with mPEG2000-MAL.

### Example 13. Detection of cell proliferation inhibition

In the present disclosure, two cell lines of breast cancer cells, SK-BR-3 and MCF-7 (purchased from Procell), were selected and a CCK8 kit (purchased from APExBIO) was used as a proliferation detection reagent to determine the inhibition effect of the following compounds on the cell proliferation: the drug molecule MMAE, the cysteine mutants of the anti-HER2 Trastuzumab single chain antibody fragment (i.e., LC46C, LC87C, HC61C-LC46, LC46C-LC87C and HC61C-LC87C), and the anti-HER2 bispecific antibody mutant BiTE-87-87, as well as the antibody-drug conjugates obtained after the conjugation of the above antibody mutants with vcMMAE. The half maximal inhibitory concentrations (IC₅₀) were calculated. Wherein, SK-BR-3 is a HER2 high-expressing cell line, and MCF-7 is a HER2 low-expressing cell line. The calculated IC₅₀ of MMAE acting on SK-BR-3 was 0.20 nM; and the calculated IC₅₀ of MMAE acting on MCF-7 was 0.35 nM. The calculated IC₅₀ of the cysteine mutants of the single chain antibody fragment LC46C, LC87C, HC61C-LC46, LC46C-LC87C and HC61C-LC87C, and the anti-HER2 bispecific antibody mutant BiTE-87-87 acting on SK-BR-3 were >1000nM. The calculated IC₅₀ of the cysteine mutants of the single chain antibody fragment LC46C, LC87C, HC61C-LC46, LC46C-LC87C and HC61C-LC87C, and the anti-HER2 bispecific antibody mutant BiTE-87-87 acting on MCF-7 were >1000nM. The calculated IC₅₀ of the antibody-drug conjugates obtained after the conjugation of the cysteine mutants of the anti-HER2 Trastuzumab single chain antibody fragment LC46C, LC87C, HC61C-LC46, LC46C-LC87C and HC61C-LC87C, and the anti-HER2 bispecific antibody mutant BiTE-87-87 with vcMMAE acting on SK-BR-3 were 23.95nM, 15.55nM, 19.76nM, 17.28nM. 13.18nM and 1.703nM. respectively. The calculated IC₅₀ of the antibody-drue conjugates obtained after the conjugation of the cysteine mutants of the anti-HER2 Trastuzumab single chain antibody fragment LC46C, LC87C, HC61C-LC46, LC46C-LC87C and HC61C-LC87C, and the anti-HER2 bispecific antibody mutant BiTE-87-87 with vcMMAE acting on MCF-7 were all greater than 50nM. The above results indicate that vcMMAE is not targeted and produces a very strong non-specific killing ability for both cell types. For the SK-BR-3 cell line with high HER2 expression, the antibodies conjugated with vcMMAE were able to exert more potent cytotoxicity compared to the single chain antibody fragments alone; whereas for the MCF-7 cell line, the antibodies conjugated with vcMMAE produced only weak cytotoxic effects due to too little HER2 expression and the insufficient antigen-mediated antibody-binding and internalisation. Compared with MMAE, the magnitude of cytotoxicity produced by the antibody-drug conjugates at the same molar concentration showed a dependence on the expression of HER2 receptor, proving that MMAE has certain targeting properties after conjugated with the cysteine mutant of the single chain antibody fragment, which can reduce the side effect of toxicity and expand the window of drug therapy.

In summary, the cysteine mutations performed at positions 12, 34, 35, 38, 44, 47, 51, 60, 61, 67, 69, 78, 79, and/or 114 of the heavy chain variable region of the single chain antibody fragment, and/or at positions 19, 21, 44, 46, 47, 48, 62, 71, 75, 78, and/or 87 of the light chain variable region, can obtain the free sulfhydryl groups for conjugation, which can be successfully conjugated with the conjugated moiety as well as the drug molecule to obtain antibody-drug conjugates, thereby making the drug targetable.

### Example 14. Screening of cell lines for Trastuzumab cysteine mutant

Examples 1-7 have verified each of the mutation sites using the anti-HER2 Trastuzumab single chain antibody fragment as an example. On this basis, the present disclosure further performed mutations on the corresponding sites of the complete antibody (*i.e.*, IgG antibody) of Trastuzumab, and verified that the mutation sites described above have universality on different forms of the antibody. Specifically, the cysteine mutations were performed on the amino acids at positions 46 and 87 of the light chain of Trastuzumab in the present application, and the mutants thereof were named as trastuzumab-46C and trastuzumab-87C, respectively. The sequence listing is shown below:

| | WT Trastuzumab | trastuzumab-46C | trastuzumab-87C |
|---|---|---|---|
| The coding sequence of the heavy chain sequence | SEQ ID NO:98 | SEQ ID NO:98 | SEQ ID NO:98 |
| The coding sequence of the light chain sequence | SEQ ID NO:99 | SEQ ID NO:102 | SEQ ID NO:104 |
| The amino acid sequence of the heavy chain | SEQ ID NO:100 | SEQ ID NO:100 | SEQ ID NO:100 |
| The amino acid sequence of the light chain | SEQ ID NO:101 | SEQ ID NO:103 | SEQ ID NO:105 |

### Example 15. Conjugation of cysteine mutants of Trastuzumab antibody with mPEG2000-MAL and detection thereof

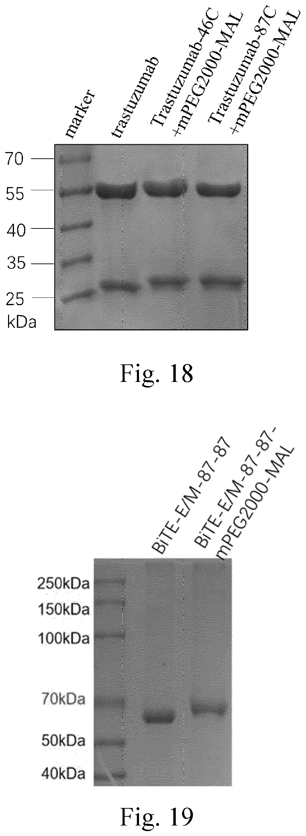

In this Example, the above cysteine mutants prepared by mutating amino acids at positions 46 and 87 of the light chain of Trastuzumab in Example 14 were used as examples, and mPEG2000-MAL (purchased from Shanghai STANDARDS Biotechnology Co., Ltd., the product identification number: ZZP-MPEG-MAL-2K-01) was used as a conjugated moiety to carry out the experiments. The conjugated reaction conditions of the antibody mutants and the conjugated moiety were: the antibody: the conjugated moiety (mPEG2000-MAL)=1:30 (molar ratio), the reaction temperature of 37°C, the reaction time of 3h, and the reaction buffer being PBS (pH 7.2). Wherein, the reduced SDS-PAGE electropherogram of the antibody mutants conjugated with mPEG2000-MAL is shown in Figure 18. The conjugation of the mutants with mPEG2000-MAL was detected using the reduced SDS-PAGE experiment. The protein bands of the light chains shifted upward after conjugation, indicating an increase in the molecular weight. The result shows that the mutants of Trastuzumab (*i.e.*, in the form of an IgG antibody) can be successfully conjugated with mPEG2000-MAL. The mutant sites also have similar technical effects in the IgG antibody form as those of the single chain antibody fragments and the bispecific antibodies.

### Example 16. Screening of the cell lines of the cysteine mutants of the anti-EGFR and anti-c-Met bispecific antibody in the form of BiTE

On the basis that Examples 9 and 11 have verified the conjugation property of the bispecific antibody in the form of BiTE constructed by the anti-HER2 Trastuzumab single chain antibody fragment and the anti-HER2 Pertuzumab single chain antibody fragment, the present disclosure further constructed a bispecific antibody in the form of BiTE based on the anti-EGFR cetuximab single chain antibody fragment and the anti-c-Met single chain antibody fragment, which was named as BiTE-E/M-WT. Moreover, the cysteine mutants of bispecific antibodies in the form of BiTE were constructed by performing the cysteine mutations at position 46 or 87 of the light chains of the anti-EGFR cetuximab single chain antibody fragment and the anti-c-Met single chain antibody fragment, respectively. For the cysteine mutants of bispecific antibodies in the form of BiTE, the names are written with the VL mutation site in the anti-EGFR cetuximab single chain antibody fragment in front of the VL mutation site in the anti-c-Met single chain antibody fragment. They were designated as BiTE-E/M-46-46, BiTE-E/M-46-87, BiTE-E/M-87-46, and BiTE-E/M-87-87, respectively. The sequences are shown in the table below.

| Name | The coding sequenc e of cetuxim ab-VH | The coding sequence of cetuximab -VL | The coding sequenc e of cetuxim ab - in-chain linker | The amino acid sequenc e of cetuxim ab -VH | The amino acid sequenc e of cetuxim ab -VL | The amino acid sequenc e of cetuxim ab - in-chain linker | The coding sequenc e of Anti-cMET-VH | The coding sequenc e of Anti-cMET - VL | The coding sequenc e of Anti-cMET-in-chain linker | The amino acid sequenc e of Anti-cMET-VH | The amino acid sequenc e of Anti-cMET-VL | The amino acid sequence of Anti-cMET - in-chain linker | The coding sequence of cetuximab - Anti-cMET interchain linker | The amino acid sequence of cetuximab - Anti-cMET interchain linker |
|---|---|---|---|---|---|---|---|---|---|---|---|---|---|---|
| BiTE-E/M-WT | SEQ ID NO:106 | SEQ ID NO:107 | SEQ ID NO:3 | SEQ ID NO:108 | SEQ ID NO:109 | SEQ ID NO:6 | SEQ ID NO:110 | SEQ ID NO:111 | SEQ ID NO:112 | SEQ ID NO:113 | SEQ ID NO:114 | SEQ ID NO:115 | SEQ ID NO:93 | SEQ ID NO:94 |
| BiTE-E/M-46-46 | SEQ ID NO:106 | SEQ ID NO:116 | SEQ ID NO:3 | SEQ ID NO:108 | SEQ ID NO:117 | SEQ ID NO:6 | SEQ ID NO:110 | SEQ ID NO:118 | SEQ ID NO:112 | SEQ ID NO:113 | SEQ ID NO:119 | SEQ ID NO:115 | SEQ ID NO:93 | SEQ ID NO:94 |
| BiTE-E/M-46-87 | SEQ ID NO:106 | SEQ ID NO:116 | SEQ ID NO:3 | SEQ ID NO:108 | SEQ ID NO:117 | SEQ ID NO:6 | SEQ ID NO:110 | SEQ ID NO:120 | SEQ ID NO:3 | SEQ ID NO:113 | SEQ ID NO:121 | SEQ ID NO:6 | SEQ ID NO:93 | SEQ ID NO:94 |
| BiTE-E/M-87-46 | SEQ ID NO:106 | SEQ ID NO:122 | SEQ ID NO:3 | SEQ ID NO:108 | SEQ ID NO:123 | SEQ ID NO:6 | SEQ ID NO:110 | SEQ ID NO:118 | SEQ ID NO:112 | SEQ ID NO:113 | SEQ ID NO:119 | SEQ ID NO:115 | SEQ ID NO:93 | SEQ ID NO:94 |
| BiTE-E/M-87-87 | SEQ ID NO:106 | SEQ ID NO:122 | SEQ ID NO:3 | SEQ ID NO:108 | SEQ ID NO:123 | SEQ ID NO:6 | SEQ ID NO:110 | SEQ ID NO:120 | SEQ ID NO:112 | SEQ ID NO:113 | SEQ ID NO:121 | SEQ ID NO:115 | SEQ ID NO:93 | SEQ ID NO:94 |

### Example 17. Conjugation of the cysteine mutants of the anti-EGFR and anti-c-Met bispecific antibody in the form of BiTE with mPEG2000-MAL and detection thereof

In this Example, the above cysteine mutants of the anti-EGFR and anti-c-Met bispecific antibodies in the form of BiTE were used as examples, and mPEG2000-MAL (purchased from Shanghai STANDARDS Biotechnology Co., Ltd., the product identification number: ZZP-MPEG-MAL-2K-01) was used as a conjugated moiety to carry out the experiments. The conjugated reaction conditions of the antibody mutants and the conjugated moiety were: the antibody: the conjugated moiety (mPEG2000-MAL)=1:30 (molar ratio), the reaction temperature of 37°C, the reaction time of 3h, and the reaction buffer being PBS (pH 7.2). Using BiTE-E/M-87-87 as an example, the conjugation of the mutant with mPEG2000-MAL was detected by the SDS-PAGE experiment. The protein bands shifted upward after conjugation, indicating an increase in the molecular weight. The result of Figure 19 shows that the mutants of the anti-EGFR and anti-c-Met bispecific antibody in the form of BiTE can be successfully conjugated with mPEG2000-MAL.

### Example 18. Screening of the cell lines of the single-site cysteine mutants of the anti-HER2 single chain bispecific antibodies in differently arranged forms

Furthermore, the present disclosure also constructed bispecific antibodies with different configurations based on the anti-HER2 Trastuzumab single chain antibody fragment and the anti-HER2 Pertuzumab single chain antibody fragment. The linkage forms and the names of the configurations are as shown in Table 5, which covers the representative cases with the linkers of different lengths, the linkage of the light and heavy chains in a different order, and the linkage of the antibodies in different orders (wherein, "Bi" denotes the same arranged order of the light and heavy chains as that in Example 9, "Tan" denotes the same arranged order of the light and heavy chains of the two antibodies, "LH"/"HL" denotes that the light chain/heavy chain comes first in the form of 'Tan', and the number at the end denotes the repeat number of the G₄S linker connecting the different antibodies).

**Table 5. Summary of the names of the anti-HER2 bispecific antibodies**

| The linking form | Name |
|---|---|
| Trastuzumab VL-(G₄S)₃-Trastuzumab VH-G₄S-Pertuzumab VH-(G₄S)₃-Pertuzumab VL | Bi-TP-1(being equivalent to BiTE-WT in Example 9) |
| Trastuzumab VL-(G₄S)₃-Trastuzumab VH-(G₄S)₂-Pertuzumab VH-(G₄S)₃-Pertuzumab VL | Bi-TP-2 |
| Trastuzumab VL-(G₄S)₃-Trastuzumab VH-(G₄S)₃-Pertuzumab VH-(G₄S)₃-Pertuzumab VL | Bi-TP-3 |
| Trastuzumab VL-(G₄S)₃-Trastuzumab VH-(G₄S)₄-Pertuzumab VH-(G₄S)₃-Pertuzumab VL | Bi-TP-4 |
| Trastuzumab VH-(G₄S)₃-Trastuzumab VL-(G₄S)₄-Pertuzumab VH-(G₄S)₃-Pertuzumab VL | Tan-HL-TP-4 |
| Trastuzumab VL-(G₄S)₃-Trastuzumab VH-(G₄S)₄-Pertuzumab Vl-(G₄S)₃-Pertuzumab VH | Tan-LH-TP-4 |
| Pertuzumab VL-(G₄S)₃-Pertuzumab VH-(G₄S)₄-Trastuzumab Vl-(G4S)₃-Trastuzumab VH | Tan-LH-PT-4 |

The coding sequences and amino acid sequences of different G₄S linkers are shown in the table below:

| The repeat number of the G₄S linker | The coding sequence of the interchain linker | The amino acid sequence of the interchain linker |
|---|---|---|
| 1 | SEQ ID NO:93 | SEQ ID NO:94 |
| 2 | SEQ ID NO:124 | SEQ ID NO:125 |
| 3 | SEQ ID NO:3 | SEQ ID NO:6 |
| 4 | SEQ ID NO:126 | SEQ ID NO:127 |

The cysteine single-site mutants of bispecific antibodies with different configurations were constructed by performing a cysteine mutation at position 46 or 87 of the light chain of the Trastuzumab single chain antibody fragment or the Pertuzumab single chain antibody fragment in the bispecific antibodies in the above table, and were named differently according to the selection of the mutant antibodies. For example, Bi-TP-1-WT-87, which has the same order of arrangement as that in Example 9, wherein the Trastuzumab was kept in the wild type, and the Pertuzumab was the one with the amino acid at position 87 of the light chain mutated to cysteine.

### Example 19. Conjugation of the single-site cysteine mutants of the anti-HER2 single chain bispecific antibodies with the small molecule drug vcMMAE and detection thereof

In this Example, the above bispecific antibodies with a single-site mutation at position 87 of Pertuzumab obtained in Example 18 was used as an example, wherein the drug molecule vcMMAE (purchased from Nanjing Adooq Bioscience Co., Ltd., the product identification number: A14362) was used as a conjugated moiety to carry out the conjugated experiments with the antibody mutants Bi-TP-1-WT-87, Bi-TP-2-WT-87, Bi-TP-3-WT-87, Bi-TP-4-WT-87, Tan-HL-TP-4-WT-87, Tan-LH-TP-4-WT-87, and Tan-LH-PT-4-87-WT. Wherein, the conjugated reaction conditions of the antibody and the conjugated moiety were: the antibody: the conjugated moiety (vcMMAE)=1:5 (molar ratio), the reaction temperature of 37°C, the reaction time of 3h, and the reaction buffer being PBS (pH 7.2).

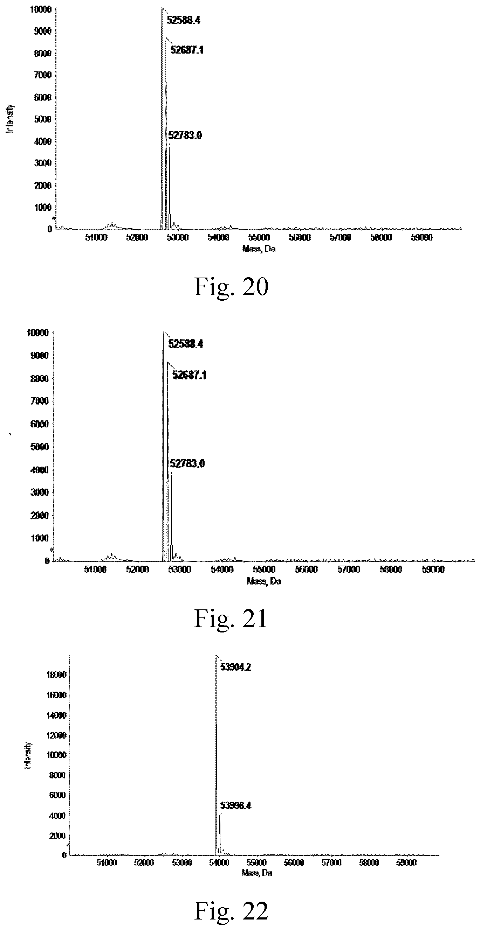

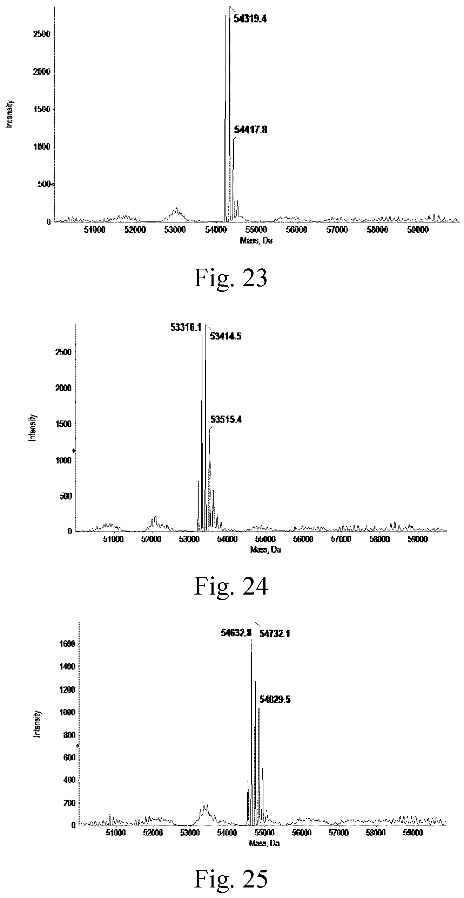

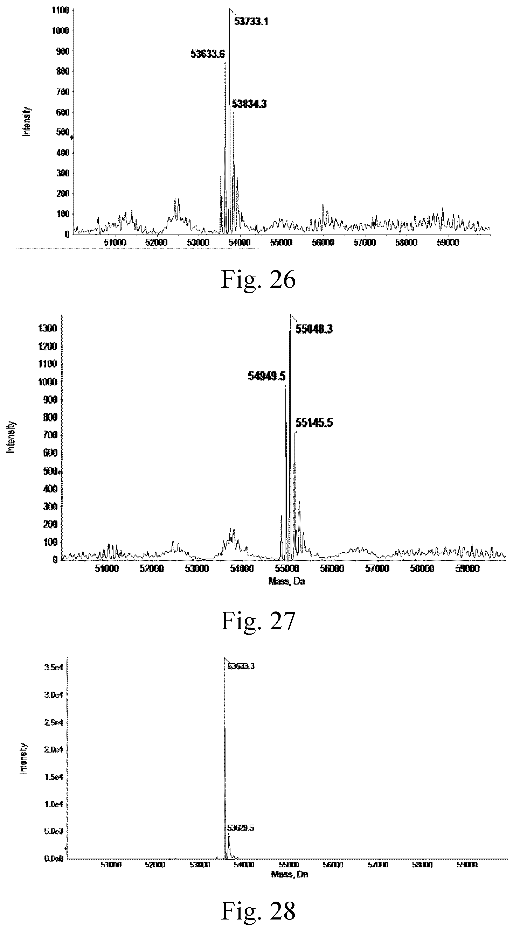

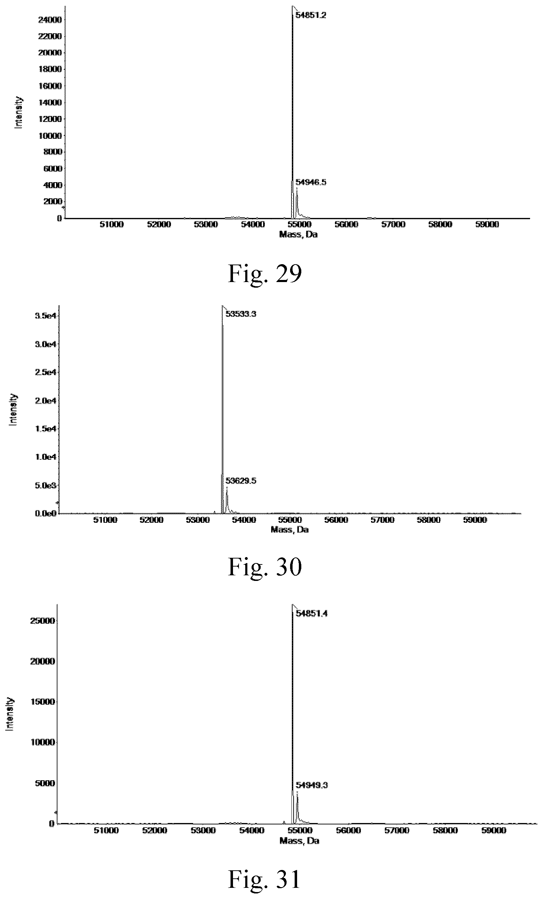

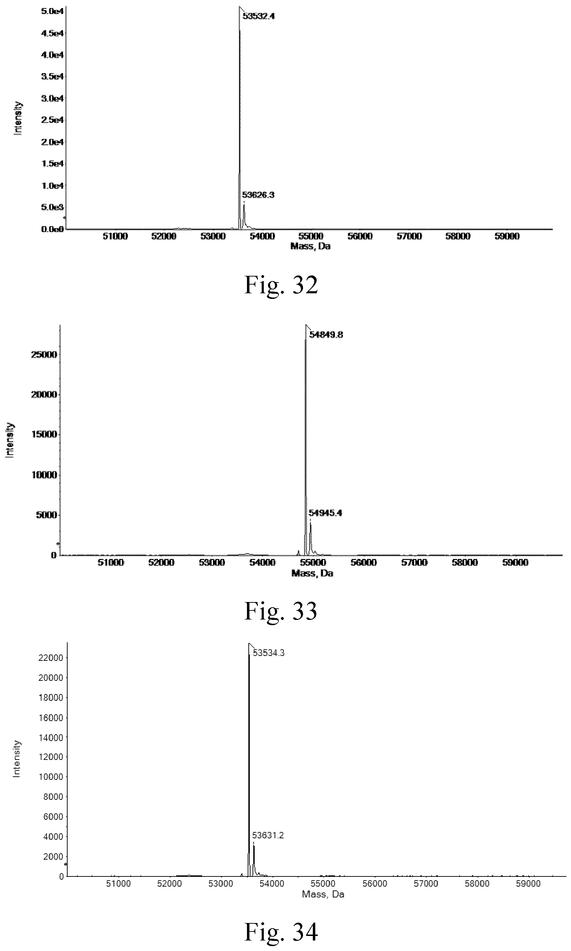

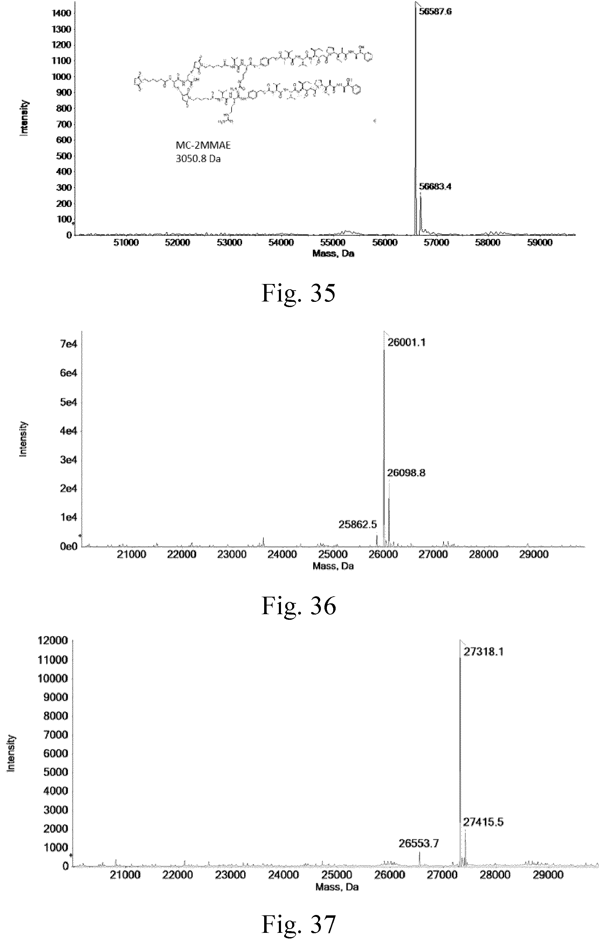

The conjugations of the single-site cysteine mutants of bispecific antibodies in differently arranged forms are shown in Figures 20-35. By comparing Figure 20 with Figure 21, Figure 22 with Figure 23, Figure 24 with Figure 25, Figure 26 with Figure 27, Figure 28 with Figure 29, Figure 30 with Figure 31, Figure 32 with Figure 33, and Figure 34 with Figure 35, respectively, it can be found that the difference in molecular weights before and after conjugation is about 1316 Da, that is, all of the single-site cysteine mutants of bispecific antibodies in differently arranged forms can be conjugated.

### Example 20. Conjugation of the antibodies with the small molecule drug MC-2MMAE and detection thereof

In this Example, the single-site cysteine mutant Bi-TP-4-WT-87 of anti-HER2 single chain bispecific antibody obtained in Example 18 was used as an example, and a dual-branching drug MC-2MMAE (purchased from Yantai MabPlex International Co., Ltd,) was used as a conjugated moiety. Wherein, the conjugated reaction conditions of the antibody and the conjugated moiety were: the antibody: the conjugated moiety (vcMMAE)=1:2.5 (molar ratio), the reaction temperature of 4°C, the reaction time of 12h, and the reaction buffer being PBS (pH 7.2). The typical diagrams before and after conjugation are shown in Figures 34-35. The molecular weight before conjugation is shown in Figure 34, which is 53534.3 Da, and the molecular weight after conjugated with the drug is shown in Figure 35, which is 56587.6 Da. The difference in molecular weights before and after conjugation is 3053.3 Da, that is, one MC-2MMAE molecule was conjugated and the same technical effect as that of Example 11 was realized. That is, the antibody mutants of the present disclosure can also be conjugated with a multibranching drug, thereby allowing for more loads to be connected through multi-branched drug linkers.

### Example 21. Screening of the cell lines of the single-site cysteine mutant of the anti-c-MET single chain antibody fragment

The mutation sites at positions 46, 87 and the like of the light chain were verified in Examples 1-8 using the Trastuzumab single chain antibody fragment, the Pertuzumab single chain antibody fragment, the Rituximab single chain antibody fragment, the Muromonab single chain antibody fragment, and the Ramucirumab single chain antibody fragment as examples. On the basis of Examples 1-8, the present disclosure also performed mutations on the anti-c-MET antibody, and verified that the above mutation sites have universality on various antibodies. Specifically, the present disclosure performed cysteine mutation at position 46 of the light chain of the anti-c-MET single chain antibody fragment, for which the mutant was named c-MET-46C. The sequence listing is shown below.

| | The coding sequence of VH | The coding sequence of VL | The sequence of the linking chain | The amino acid sequence of VH | The amino acid sequence of VL | The amino acid sequence of the linking chain |
|---|---|---|---|---|---|---|
| c-MET-46C | SEQ ID NO:110 | SEQ ID NO:118 | SEQ ID NO:3 | SEQ ID **NO:113** | SEQ ID NO:119 | SEQ ID NO:6 |

### Example 22. Conjugation of the single-site cysteine mutant of the anti-c-MET single chain antibody fragment with vcMMAE and detection thereof

In this Example, the position 46 single-site mutant of anti-c-MET single chain antibody fragment prepared in Example 21 was used as an example, and the drug molecule vcMMAE (purchased from Nanjing Adooq Bioscience Co, Ltd., the product identification number: A14362) was used as a conjugated moiety to carry out the conjugated experiments. Wherein, the conjugated reaction conditions of the antibody and the conjugated moiety were: the antibody: the conjugated moiety (vcMMAE)=1:5 (molar ratio), the reaction temperature of 37°C, the reaction time of 3h, and the reaction buffer being PBS (pH 7.2).

The diagrams before and after conjugation are shown in Figures 36 and 37, wherein Figure 36 is a result image showing the molecular weight of the single chain antibody fragment before conjugation, with the molecular weight being 26001.1 Da; and Figure 37 is a result image showing the molecular weight of the antibody after conjugated with vcMMAE, with the molecular weight of the antibody after conjugated with vcMMAE being 27318.1 Da, and the calculated difference in molecular weight is the molecular weight of 1 vcMMAE, that is, each antibody mutant can be successfully conjugated with one vcMMAE.

### Example 22. Screening of the cell lines of the single-site cysteine mutant of the anti-EGFR single chain antibody fragment

The mutation sites at positions 46, 87 and the like of different single chain antibody fragments were verified in Examples 1-8 and 21. On this basis, the present disclosure also performed mutation at position 46 of the light chain of an anti-EGFR Zalutumumab single chain antibody fragment, and verified that the above mutation site has universality on various antibodies. Specifically, the present disclosure performed cysteine mutation at position 46 of the light chain of the Zalutumumab single chain antibody fragment, for which the mutant was named as Zalu-46C. The sequence listing is shown below.

| | The coding sequence of VH | The coding sequence of VL | The sequence of the linking chain | The amino acid sequence of VH | The amino acid sequence of VL | The amino acid sequence of the linking chain |
|---|---|---|---|---|---|---|
| c-MET-46C | SEQ ID NO: 128 | SEQ ID NO: 129 | SEQ ID NO:3 | SEQ ID NO:130 | SEQ ID NO:131 | SEQ ID NO:6 |

### Example 23. Conjugation of the single-site cysteine mutant of the anti-EGFR single chain antibody fragment with vcMMAE and detection thereof

In this Example, the position 46 single-site mutant of Zalutumumab single chain antibody fragment prepared in Example 22 was used as an example, and the drug molecule vcMMAE (purchased from Nanjing Adooq Bioscience Co., Ltd., the product identification number: A14362) was used as a conjugated moiety to carry out the conjugated experiments. Wherein, the conjugated reaction conditions of the antibody and the conjugated moiety were: the antibody: the conjugated moiety (vcMMAE)=1:5 (molar ratio), the reaction temperature of 37°C, the reaction time of 3h, and the reaction buffer being PBS (pH 7.2).

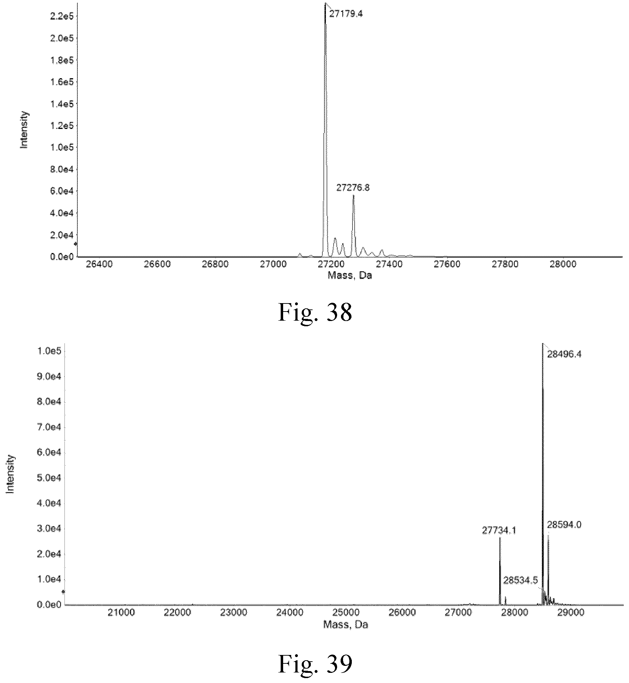

The diagrams before and after conjugation are shown in Figures 38 and 39, wherein Figure 38 is a result image showing the molecular weight of the single chain antibody fragment before conjugation, with the molecular weight being 27179.4 Da; and Figure 39 is a result image showing the molecular weight of the antibody after conjugated with vcMMAE, with the molecular weight of the antibody after conjugated with vcMMAE being 28496.4 Da, and the calculated difference in molecular weight is the molecular weight of 1 vcMMAE, that is, each antibody mutant can be successfully conjugated with one vcMMAE.

The above mentioned specific examples further illustrate in detail the purposes, technical solutions and beneficial effects of the present disclosure. It should be understood that the above mentioned examples are only specific examples of the present disclosure, and are not intended to be used to limit the present disclosure. Any modifications, equivalent substitutions, improvements, etc. made within the spirit and principles of the present disclosure should be encompassed in the scope of the present disclosure.

### REFERENCES

1. A Potent Anti-CD70 Antibody-Drug Conjugate Combining a DimericPyrrolobenzodiazepine Drug with Site-Specific Conjugation Technology [J]. Bioconjugate Chemistry, 2013, 24(7):1256-1263.
2. Crivianu-Gaita V, Thompson M. Aptamers, antibody scFv, and antibody Fab0 fragments: an overview and comparison of three of the most versatile biosensor biorecognition elements. BiosensBioelectron 2016; 85:32-45.
3. Ahmad ZA, Yeap SK, Ali AM, Ho WY, Alitheen NBM, Hamid M. ScFv antibody: principles and clinical application. Clin Dev Immunol 2012; 2012. Article ID 980250.
4. Safdari Y, Ahmadzadeh V. Use of single-chain antibody derivatives for targeted drug delivery. Mol Med 2016; 22:258-70.
5. Deonarain MP. Fragment antibody fragment drug conjugates (FDCs): a unique drug class or just smaller ADCs?In: Proceedings of the protein engineering summit (PEGS) conference; 2017.
6.Deonarain M, Yahioglu G, Stamati I, Pomowski A, Clarke J, Edwards B, et al. Small-format drug conjugates: a viable alternative to ADCs for solid tumours? Antibodies 2018; 7:16.
7. Deonarain MP. Fragment antibody fragment drug conjugates (FDCs): a unique drug class or just smaller ADCs?In: Proceedings of the Protein Engineering Summit (PEGS) Conference; 2017.

### SEQUENCE LISTING

SEQ ID NO:1 
GAAGTGCAGCTGGTGGAATCCGGAGGAGGACTGGTGCAACCTGGAGGGTCCCTGAGG
TTGTCTTGTGCTGCCTCCGGCTTCAACATCAAGGACACCTACATCCACTGGGTGAGGC
AAGCTCCTGGCAAAGGACTGGAATGGGTGGCTAGGATCTATCCCACCAACGGCTACAC
AAGGTATGCCGACTCCGTGAAGGGAAGGTTCACCATCTCTGCCGACACCTCCAAGAAT
ACCGCCTACCTGCAGATGAATTCCCTGAGGGCCGAAGACACAGCTGTGTACTACTGCT
CCAGGTGGGGCGGAGATGGCTTTTACGCCATGGACTACTGGGGACAGGGAACACTGG
TGACCGTTTCCTCC
SEQ ID NO:2 
GATATCCAGATGACCCAGTCCCCTTCTTCCCTGTCCGCTTCTGTGGGAGACAGGGTGAC
AATCACCTGTAGGGCCTCCCAGGACGTGAATACAGCTGTGGCCTGGTACCAACAAAAG
CCCGGCAAAGCTCCCAAACTGCTGATTTACTCCGCCTCCTTCCTGTATTCTGGAGTGCC
CTCCAGGTTTTCTGGGTCCAGGTCCGGAACAGACTTCACACTGACCATCTCCTCCCTG
CAGCCTGAAGACTTCGCCACCTACTACTGCCAGCAGCACTATACCACCCCCCCTACATT
TGGACAAGGCACCAAGGTGGAGATTAAG
SEQ ID NO:3
   GGAGGAGGAGGATCTGGAGGAGGAGGATCTGGAGGAGGAGGAAGC
SEQ ID NO:4
SEQ ID NO:5 


SEQ ID NO:6：GGGGSGGGGSGGGGS
SEQ ID NO:7 GAAGTGCAGCTGGTGGAATCCGGAGGAGGACTGTGTCAACCTGGAGGGTCCCTGAGG 
TTGTCTTGTGCTGCCTCCGGCTTCAACATCAAGGACACCTACATCCACTGGGTGAGGC
AAGCTCCTGGCAAAGGACTGGAATGGGTGGCTAGGATCTATCCCACCAACGGCTACAC
AAGGTATGCCGACTCCGTGAAGGGAAGGTTCACCATCTCTGCCGACACCTCCAAGAAT
ACCGCCTACCTGCAGATGAATTCCCTGAGGGCCGAAGACACAGCTGTGTACTACTGCT
CCAGGTGGGGCGGAGATGGCTTTTACGCCATGGACTACTGGGGACAGGGAACACTGG
TGACCGTTTCCTCC
SEQ ID NO:8 
EVQLVESGGGLCQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SS
SEQ ID NO:9 
GAAGTGCAGCTGGTGGAATCCGGAGGAGGACTGGTGCAACCTGGAGGGTCCCTGAGG
TTGTCTTGTGCTGCCTCCGGCTTCAACATCAAGGACACCTACATCTGTTGGGTGAGGCA
AGCTCCTGGCAAAGGACTGGAATGGGTGGCTAGGATCTATCCCACCAACGGCTACACA
AGGTATGCCGACTCCGTGAAGGGAAGGTTCACCATCTCTGCCGACACCTCCAAGAATA
CCGCCTACCTGCAGATGAATTCCCTGAGGGCCGAAGACACAGCTGTGTACTACTGCTC
CAGGTGGGGCGGAGATGGCTTTTACGCCATGGACTACTGGGGACAGGGAACACTGGT
GACCGTTTCCTCC
SEQ ID NO:10 
EVQLVESGGGLVQPGGSLRLSCAASGFNIKDTYICWVRQAPGKGLEWVARIYPTNGYTRY
ADSVKGRFTISADTSKNTAYLQMNSLRAEDTAVYYCSRWGGDGFYAMDYWGQGTLVTV
SS
SEQ ID NO:11 
GAAGTGCAGCTGGTGGAATCCGGAGGAGGACTGGTGCAACCTGGAGGGTCCCTGAGG
TTGTCTTGTGCTGCCTCCGGCTTCAACATCAAGGACACCTACATCCACTGGGTGAGGC
AAGCTCCTGGCAAAGGACTGGAATGGGTGGCTAGGATCTATCCCACCAACGGCTACAC
AAGGTATTGTGACTCCGTGAAGGGAAGGTTCACCATCTCTGCCGACACCTCCAAGAAT
ACCGCCTACCTGCAGATGAATTCCCTGAGGGCCGAAGACACAGCTGTGTACTACTGCT
CCAGGTGGGGCGGAGATGGCTTTTACGCCATGGACTACTGGGGACAGGGAACACTGG
TGACCGTTTCCTCC
SEQ ID NO:12 
EVQLVESGGGLVQPGGSLRLSCAASGFNIKDTYIHWVRQAPGKGLEWVARIYPTNGYTRY
CDSVKGRFTISADTSKNTAYLQMNSLRAEDTAVYYCSRWGGDGFYAMDYWGQGTLVTV
SS
SEQ ID NO:13 
GATATCCAGATGACCCAGTCCCCTTCTTCCCTGTCCGCTTCTGTGGGAGACAGGGTGAC
ATGTACCTGTAGGGCCTCCCAGGACGTGAATACAGCTGTGGCCTGGTACCAACAAAAG
CCCGGCAAAGCTCCCAAACTGCTGATTTACTCCGCCTCCTTCCTGTATTCTGGAGTGCC
CTCCAGGTTTTCTGGGTCCAGGTCCGGAACAGACTTCACACTGACCATCTCCTCCCTG
CAGCCTGAAGACTTCGCCACCTACTACTGCCAGCAGCACTATACCACCCCCCCTACATT
TGGACAAGGCACCAAGGTGGAGATTAAG
SEQ ID NO:14 
DIQMTQSPSSLSASVGDRVTCTCRASQDVNTAVAWYQQKPGKAPKLLIYSASFLYSGVPSR
FSGSRSGTDFTLTISSLQPEDFATYYCQQHYTTPPTFGQGTKVEIK
SEQ ID NO:15 
GATATCCAGATGACCCAGTCCCCTTCTTCCCTGTCCGCTTCTGTGGGAGACAGGGTGAC
AATCACCTGTAGGGCCTCCCAGGACGTGAATACAGCTGTGGCCTGGTACCAACAAAAG
CCCGGCAAAGCTCCCAAACTGTGTATTTACTCCGCCTCCTTCCTGTATTCTGGAGTGCC
CTCCAGGTTTTCTGGGTCCAGGTCCGGAACAGACTTCACACTGACCATCTCCTCCCTG
CAGCCTGAAGACTTCGCCACCTACTACTGCCAGCAGCACTATACCACCCCCCCTACATT
TGGACAAGGCACCAAGGTGGAGATTAAG
SEQ ID NO:16 
DIQMTQSPSSLSASVGDRVTITCRASQDVNTAVAWYQQKPGKAPKLCIYSASFLYSGVPSR
FSGSRSGTDFTLTISSLQPEDFATYYCQQHYTTPPTFGQGTKVEIK
SEQ ID NO:17 
GATATCCAGATGACCCAGTCCCCTTCTTCCCTGTCCGCTTCTGTGGGAGACAGGGTGAC
AATCACCTGTAGGGCCTCCCAGGACGTGAATACAGCTGTGGCCTGGTACCAACAAAAG
CCCGGCAAAGCTCCCAAACTGCTGTGTTACTCCGCCTCCTTCCTGTATTCTGGAGTGCC
CTCCAGGTTTTCTGGGTCCAGGTCCGGAACAGACTTCACACTGACCATCTCCTCCCTG
CAGCCTGAAGACTTCGCCACCTACTACTGCCAGCAGCACTATACCACCCCCCCTACATT
TGGACAAGGCACCAAGGTGGAGATTAAG
SEQ ID NO:18 
DIQMTQSPSSLSASVGDRVTITCRASQDVNTAVAWYQQKPGKAPKLLCYSASFLYSGVPSR
FSGSRSGTDFTLTISSLQPEDFATYYCQQHYTTPPTFGQGTKVEIK
SEQ ID NO:19 
GATATCCAGATGACCCAGTCCCCTTCTTCCCTGTCCGCTTCTGTGGGAGACAGGGTGAC
AATCACCTGTAGGGCCTCCCAGGACGTGAATACAGCTGTGGCCTGGTACCAACAAAAG
CCCGGCAAAGCTCCCAAACTGCTGATTTACTCCGCCTCCTTCCTGTATTCTGGAGTGCC
 
CTCCAGGTTTTCTGGGTCCAGGTCCGGAACAGACTGTACACTGACCATCTCCTCCCTG
CAGCCTGAAGACTTCGCCACCTACTACTGCCAGCAGCACTATACCACCCCCCCTACATT
TGGACAAGGCACCAAGGTGGAGATTAAG
SEQ ID NO:20 
DIQMTQSPSSLSASVGDRVTITCRASQDVNTAVAWYQQKPGKAPKLLIYSASFLYSGVPSR
FSGSRSGTDCTLTISSLQPEDFATYYCQQHYTTPPTFGQGTKVEIK
SEQ ID NO:21 
GATATCCAGATGACCCAGTCCCCTTCTTCCCTGTCCGCTTCTGTGGGAGACAGGGTGAC
AATCACCTGTAGGGCCTCCCAGGACGTGAATACAGCTGTGGCCTGGTACCAACAAAAG
CCCGGCAAAGCTCCCAAACTGCTGATTTACTCCGCCTCCTTCCTGTATTCTGGAGTGCC
CTCCAGGTTTTCTGGGTCCAGGTCCGGAACAGACTTCACACTGACCTGTTCCTCCCTG
CAGCCTGAAGACTTCGCCACCTACTACTGCCAGCAGCACTATACCACCCCCCCTACATT
TGGACAAGGCACCAAGGTGGAGATTAAG
SEQ ID NO:22 
DIQMTQSPSSLSASVGDRVTITCRASQDVNTAVAWYQQKPGKAPKLLIYSASFLYSGVPSR
FSGSRSGTDFTLTCSSLQPEDFATYYCQQHYTTPPTFGQGTKVEIK
HC 34C
SEQ ID NO :23 
gaagtgcagc tggtggaatc cggaggagga ctggtgcaac ctggagggtc cctgaggttg tcttgtgctg cctccggctt
caacatcaag gacacctact gtcactgggt gaggcaagct cctggcaaag gactggaatg ggtggctagg atctatccca
ccaacggcta cacaaggtat gccgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :24 
EVQLVESGGGLVQPGGSLRLSCAASGFNIKDTYCHWVRQAPGKGLEWVARIYPTNGYTR
YADSVKGRFTISADTSKNTAYLQMNSLRAEDTAVYYCSRWGGDGFYAMDYWGQGTLVT
VSS
HC 38C
SEQ ID NO :25 
gaagtgcagc tggtggaatc cggaggagga ctggtgcaac ctggagggtc cctgaggttg tcttgtgctg cctccggctt
caacatcaag gacacctaca tccactgggt gtgtcaagct cctggcaaag gactggaatg ggtggctagg atctatccca
ccaacggcta cacaaggtat gccgactccg tgaagggaag gttcaccatc tctgccgaca cctccaagaa taccgcctac
 
ctgcagatga attccctgag ggccgaagac acagctgtgt actactgctc caggtggggc ggagatggct tttacgccat
ggactactgg ggacagggaa cactggtgac cgtttcctcc
SEQ ID NO :26 
EVQLVESGGGLVQPGGSLRLSCAASGFNIKDTYIHWVCQAPGKGLEWVARIYPTNGYTRY
ADSVKGRFTISADTSKNTAYLQMNSLRAEDTAVYYCSRWGGDGFYAMDYWGQGTLVTV
SS
HC 44C
SEQ ID NO :27 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaat gtctggaatg ggtggctagg atctatccca
ccaacggcta cacaaggtat gccgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :28 
EVQLVESGGGLVQPGGSLRLSCAASGFNIKDTYIHWVRQAPGKCLEWVARIYPTNGYTRY
ADSVKGRFTISADTSKNTAYLQMNSLRAEDTAVYYCSRWGGDGFYAMDYWGQGTLVTV
SS
HC 47C
SEQ ID NO :29 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg tgtggctagg atctatccca
ccaacggcta cacaaggtat gccgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :30 
EVQLVESGGGLVQPGGSLRLSCAASGFNIKDTYIHWVRQAPGKGLECVARIYPTNGYTRY
ADSVKGRFTISADTSKNTAYLQMNSLRAEDTAVYYCSRWGGDGFYAMDYWGQGTLVTV
SS
HC 51C
SEQ ID NO :31 GAAGTGCAGCTGGTGGAATCCGGAGGAGGACTGGTGCAACCTGGAGGGTCCCTGAGG 
TTGTCTTGTGCTGCCTCCGGCTTCAACATCAAGGACACCTACATCCACTGGGTGAGGC
AAGCTCCTGGCAAAGGACTGGAATGGGTGGCTAGGTGTTATCCCACCAACGGCTACAC
AAGGTATGCCGACTCCGTGAAGGGAAGGTTCACCATCTCTGCCGACACCTCCAAGAAT
ACCGCCTACCTGCAGATGAATTCCCTGAGGGCCGAAGACACAGCTGTGTACTACTGCT
CCAGGTGGGGCGGAGATGGCTTTTACGCCATGGACTACTGGGGACAGGGAACACTGG
TGACCGTTTCCTCC
SEQ ID NO :32 
EVQLVESGGGLVQPGGSLRLSCAASGFNIKDTYIHWVRQAPGKGLEWVARCYPTNGYTR
YADSVKGRFTISADTSKNTAYLQMNSLRAEDTAVYYCSRWGGDGFYAMDYWGQGTLVT
VSS
HC 60C
SEQ ID NO :33 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtgt gccgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :34 
EVQLVESGGGLVQPGGSLRLSCAASGFNIKDTYIHWVRQAPGKGLEWVARIYPTNGYTRC
ADSVKGRFTISADTSKNTAYLQMNSLRAEDTAVYYCSRWGGDGFYAMDYWGQGTLVTV
SS
HC 67C
SEQ ID NO :35 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtat gccgactccg tgaagggatg tttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :36 
EVQLVESGGGLVQPGGSLRLSCAASGFNIKDTYIHWVRQAPGKGLEWVARIYPTNGYTRY
ADSVKGCFTISADTSKNTAYLQMNSLRAEDTAVYYCSRWGGDGFYAMDYWGQGTLVTV
SS
HC 69C
SEQ ID NO :37 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtat gccgactccg tgaagggaag gttctgtatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :38 
EVQLVESGGGLVQPGGSLRLSCAASGFNIKDTYIHWVRQAPGKGLEWVARIYPTNGYTRY
ADSVKGRFCISADTSKNTAYLQMNSLRAEDTAVYYCSRWGGDGFYAMDYWGQGTLVTV
SS
HC 78C
SEQ ID NO :39 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtat gccgactccg tgaagggaag gttcaccatc tctgccgaca cctccaagaa ttgtgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :40 
EVQLVESGGGLVQPGGSLRLSCAASGFNIKDTYIHWVRQAPGKGLEWVARIYPTNGYTRY
ADSVKGRFTISADTSKNCAYLQMNSLRAEDTAVYYCSRWGGDGFYAMDYWGQGTLVTV
SS
HC 79C
SEQ ID NO :41 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtat gccgactccg tgaagggaag gttcaccatc tctgccgaca cctccaagaa tacctgttac
ctgcagatga attccctgag ggccgaagac acagctgtgt actactgctc caggtggggc ggagatggct tttacgccat
ggactactgg ggacagggaa cactggtgac cgtttcctcc
SEQ ID NO :42 
EVQLVESGGGLVQPGGSLRLSCAASGFNIKDTYIHWVRQAPGKGLEWVARIYPTNGYTRY
ADSVKGRFTISADTSKNTCYLQMNSLRAEDTAVYYCSRWGGDGFYAMDYWGQGTLVTV
 SS
HC 114C
SEQ ID NO :43 
gaagtgcagc tggtggaatc cggaggagga ctggtgcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtat gccgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggat gtctggtgac cgtttcctcc
SEQ ID NO :44 
EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGCLVTV
SS
LC 19C
SEQ ID NO :45 
gatatccaga tgacccagtc cccttcttcc ctgtccgctt ctgtgggaga caggtgtaca atcacctgta gggcctccca ggacgtgaat
acagctgtgg cctggtacca acaaaagccc ggcaaagctc ccaaactgct gatttactcc gcctccttcc tgtattctgg agtgccctcc
aggttttctg ggtccaggtc cggaacagac ttcacactga ccatctcctc cctgcagcct gaagacttcg ccacctacta
ctgccagcag cactatacca ccccccctac atttggacaa ggcaccaagg tggagattaa g
SEQ ID NO :46 
DIQMTQSPSSLSASVGDRCTITCRASQDVNTAVAWYQQKPGKAPKLLIYSASFLYSGVPS
RFSGSRSGTDFTLTISSLQPEDFATYYCQQHYTTPPTFGQGTKVEIK
LC 44C
SEQ ID NO :47 
gatatccaga tgacccagtc cccttcttcc ctgtccgctt ctgtgggaga cagggtgaca atcacctgta gggcctccca
ggacgtgaat acagctgtgg cctggtacca acaaaagccc ggcaaagctt gtaaactgct gatttactcc gcctccttcc tgtattctgg
agtgccctcc aggttttctg ggtccaggtc cggaacagac ttcacactga ccatctcctc cctgcagcct gaagacttcg ccacctacta
ctgccagcag cactatacca ccccccctac atttggacaa ggcaccaagg tggagattaa g
SEQ ID NO :48 
DIQMTQSPSSLSASVGDRVTITCRASQDVNTAVAWYQQKPGKACKLLIYSASFLYSGVPS
RFSGSRSGTDFTLTISSLQPEDFATYYCQQHYTTPPTFGQGTKVEIK
LC 46C
SEQ ID NO :49 
gatatccaga tgacccagtc cccttcttcc ctgtccgctt ctgtgggaga cagggtgaca atcacctgta gggcctccca ggacgtgaat
acagctgtgg cctggtacca acaaaagccc ggcaaagctc ccaaatgtct gatttactcc gcctccttcc tgtattctgg agtgccctcc
aggttttctg ggtccaggtc cggaacagac ttcacactga ccatctcctc cctgcagcct gaagacttcg ccacctacta ctgccagcag
cactatacca ccccccctac atttggacaa ggcaccaagg tggagattaa g
SEQ ID NO :50 
DIQMTQSPSSLSASVGDRVTITCRASQDVNTAVAWYQQKPGKAPKCLIYSASFLYSGVPS
RFSGSRSGTDFTLTISSLQPEDFATYYCQQHYTTPPTFGQGTKVEIK
LC 62C
SEQ ID NO : 51 
gatatccaga tgacccagtc cccttcttcc ctgtccgctt ctgtgggaga cagggtgaca atcacctgta gggcctccca ggacgtgaat
acagctgtgg cctggtacca acaaaagccc ggcaaagctc ccaaactgct gatttactcc gcctccttcc tgtattctgg agtgccctcc
aggtgttctg ggtccaggtc cggaacagac ttcacactga ccatctcctc cctgcagcct gaagacttcg ccacctacta ctgccagcag
cactatacca ccccccctac atttggacaa ggcaccaagg tggagattaa g
SEQ ID NO :52 
DIQMTQSPSSLSASVGDRVTITCRASQDVNTAVAWYQQKPGKAPKLLIYSASFLYSGVPS
RCSGSRSGTDFTLTISSLQPEDFATYYCQQHYTTPPTFGQGTKVEIK
LC 78C
SEQ ID NO :53 
gatatccaga tgacccagtc cccttcttcc ctgtccgctt ctgtgggaga cagggtgaca atcacctgta gggcctccca ggacgtgaat
acagctgtgg cctggtacca acaaaagccc ggcaaagctc ccaaactgct gatttactcc gcctccttcc tgtattctgg agtgccctcc
aggttttctg ggtccaggtc cggaacagac ttcacactga ccatctcctc ctgtcagcct gaagacttcg ccacctacta ctgccagcag
cactatacca ccccccctac atttggacaa ggcaccaagg tggagattaa g
SEQ ID NO :54 
DIQMTQSPSSLSASVGDRVTITCRASQDVNTAVAWYQQKPGKAPKLLIYSASFLYSGVPS
RFSGSRSGTDFTLTISSCQPEDFATYYCQQHYTTPPTFGQGTKVEIK
LC 87C
SEQ ID NO :55 
gatatccaga tgacccagtc cccttcttcc ctgtccgctt ctgtgggaga cagggtgaca atcacctgta gggcctccca
ggacgtgaat acagctgtgg cctggtacca acaaaagccc ggcaaagctc ccaaactgct gatttactcc gcctccttcc tgtattctgg
 
agtgccctcc aggttttctg ggtccaggtc cggaacagac ttcacactga ccatctcctc cctgcagcct gaagacttcg ccacctactg
ttgccagcag cactatacca ccccccctac atttggacaa ggcaccaagg tggagattaa g
SEQ ID NO :56 
DIQMTQSPSSLSASVGDRVTITCRASQDVNTAVAWYQQKPGKAPKLLIYSASFLYSGVPS
RFSGSRSGTDFTLTISSLQPEDFATYCCQQHYTTPPTFGQGTKVEIK
12-35C
SEQ ID NO :57 
gaagtgcagc tggtggaatc cggaggagga ctgtgtcaac ctggagggtc cctgaggttg tcttgtgctg cctccggctt
caacatcaag gacacctaca tctgttgggt gaggcaagct cctggcaaag gactggaatg ggtggctagg atctatccca
ccaacggcta cacaaggtat gccgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :58 
EVQLVESGGGLCQPGGSLRLSCAASGFNIKDTYICWVRQAPGKGLEWVARIYPTNGYTRY
ADSVKGRFTISADTSKNTAYLQMNSLRAEDTAVYYCSRWGGDGFYAMDYWGQGTLVTV
SS
12-61C
SEQ ID NO :59 
gaagtgcagc tggtggaatc cggaggagga ctgtgtcaac ctggagggtc cctgaggttg tcttgtgctg cctccggctt
caacatcaag gacacctaca tccactgggt gaggcaagct cctggcaaag gactggaatg ggtggctagg atctatccca
ccaacggcta cacaaggtat tgtgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :60 
EVQLVESGGGLCQPGGSLRLSCAASGFNIKDTYIHWVRQAPGKGLEWVARIYPTNGYTRY
CDSVKGRFTISADTSKNTAYLQMNSLRAEDTAVYYCSRWGGDGFYAMDYWGQGTLVTV
SS
35-61C
SEQ ID NO : 61 
gaagtgcagc tggtggaatc cggaggagga ctggtgcaac ctggagggtc cctgaggttg tcttgtgctg cctccggctt
caacatcaag gacacctaca tctgttgggt gaggcaagct cctggcaaag gactggaatg ggtggctagg atctatccca
 
ccaacggcta cacaaggtat tgtgactccg tgaagggaag gttcaccatc tctgccgaca cctccaagaa taccgcctac
ctgcagatga attccctgag ggccgaagac acagctgtgt actactgctc caggtggggc ggagatggct tttacgccat
ggactactgg ggacagggaa cactggtgac cgtttcctcc
SEQ ID NO :62 
EVQLVESGGGLVQPGGSLRLSCAASGFNIKDTYICWVRQAPGKGLEWVARIYPTNGYTRY
CDSVKGRFTISADTSKNTAYLQMNSLRAEDTAVYYCSRWGGDGFYAMDYWGQGTLVTV
SS
51-61C
SEQ ID NO :63 
GAAGTGCAGCTGGTGGAATCCGGAGGAGGACTGGTGCAACCTGGAGGGTCCCTGAGG
TTGTCTTGTGCTGCCTCCGGCTTCAACATCAAGGACACCTACATCCACTGGGTGAGGC
AAGCTCCTGGCAAAGGACTGGAATGGGTGGCTAGGTGTTATCCCACCAACGGCTACAC
AAGGTATTGTGACTCCGTGAAGGGAAGGTTCACCATCTCTGCCGACACCTCCAAGAAT
ACCGCCTACCTGCAGATGAATTCCCTGAGGGCCGAAGACACAGCTGTGTACTACTGCT
CCAGGTGGGGCGGAGATGGCTTTTACGCCATGGACTACTGGGGACAGGGAACACTGG
TGACCGTTTCCTCC
SEQ ID NO :64 
EVQLVESGGGLVQPGGSLRLSCAASGFNIKDTYIHWVRQAPGKGLEWVARCYPTNGYTR
YCDSVKGRFTISADTSKNTAYLQMNSLRAEDTAVYYCSRWGGDGFYAMDYWGQGTLVT
VSS
21-47C
SEQ ID NO :65 
gatatccaga tgacccagtc cccttcttcc ctgtccgctt ctgtgggaga cagggtgaca tgtacctgta gggcctccca ggacgtgaat
acagctgtgg cctggtacca acaaaagccc ggcaaagctc ccaaactgtg tatttactcc gcctccttcc tgtattctgg agtgccctcc
aggttttctg ggtccaggtc cggaacagac ttcacactga ccatctcctc cctgcagcct gaagacttcg ccacctacta ctgccagcag
cactatacca ccccccctac atttggacaa ggcaccaagg tggagattaa g
SEQ ID NO :66 
DIQMTQSPSSLSASVGDRVTCTCRASQDVNTAVAWYQQKPGKAPKLCIYSASFLYSGVPSR
FSGSRSGTDFTLTISSLQPEDFATYYCQQHYTTPPTFGQGTKVEIK
21-48C
SEQ ID NO :67 
gatatccaga tgacccagtc cccttcttcc ctgtccgctt ctgtgggaga cagggtgaca tgtacctgta gggcctccca ggacgtgaat
acagctgtgg cctggtacca acaaaagccc ggcaaagctc ccaaactgct gtgttactcc gcctccttcc tgtattctgg agtgccctcc
aggttttctg ggtccaggtc cggaacagac ttcacactga ccatctcctc cctgcagcct gaagacttcg ccacctacta ctgccagcag
cactatacca ccccccctac atttggacaa ggcaccaagg tggagattaa g
SEQ ID NO :68 
DIQMTQSPSSLSASVGDRVTCTCRASQDVNTAVAWYQQKPGKAPKLLCYSASFLYSGVPS
RFSGSRSGTDFTLTISSLQPEDFATYYCQQHYTTPPTFGQGTKVEIK
21-75C
SEQ ID NO :69 
gatatccaga tgacccagtc cccttcttcc ctgtccgctt ctgtgggaga cagggtgaca tgtacctgta gggcctccca ggacgtgaat
acagctgtgg cctggtacca acaaaagccc ggcaaagctc ccaaactgct gatttactcc gcctccttcc tgtattctgg agtgccctcc
aggttttctg ggtccaggtc cggaacagac ttcacactga cctgttcctc cctgcagcct gaagacttcg ccacctacta ctgccagcag
cactatacca ccccccctac atttggacaa ggcaccaagg tggagattaa g
SEQ ID NO :70 
DIQMTQSPSSLSASVGDRVTCTCRASQDVNTAVAWYQQKPGKAPKLLIYSASFLYSGVPS
RFSGSRSGTDFTLTCSSLQPEDFATYYCQQHYTTPPTFGQGTKVEIK
46-87C
SEQ ID NO : 71 
gatatccaga tgacccagtc cccttcttcc ctgtccgctt ctgtgggaga cagggtgaca atcacctgta gggcctccca ggacgtgaat
acagctgtgg cctggtacca acaaaagccc ggcaaagctc ccaaatgtct gatttactcc gcctccttcc tgtattctgg agtgccctcc
aggttttctg ggtccaggtc cggaacagac ttcacactga ccatctcctc cctgcagcct gaagacttcg ccacctactg
ttgccagcag cactatacca ccccccctac atttggacaa ggcaccaagg tggagattaa g
SEQ ID NO :72 
DIQMTQSPSSLSASVGDRVTITCRASQDVNTAVAWYQQKPGKAPKCLIYSASFLYSGVPS
RFSGSRSGTDFTLTISSLQPEDFATYCCQQHYTTPPTFGQGTKVEIK
Pertuzumab scFv-46C
SEQ ID NO :73 
gaggtgcagc tggtggagag cggcggcggc ctggtgcagc ccggcggcag cctgcgcctg agctgcgccg ccagcggctt
caccttcacc gactacacca tggactgggt gcgccaggcc cccggcaagg gcctggagtg ggtggccgac gtgaacccca
acagcggcgg cagcatctac aaccagcgct tcaagggccg cttcaccctg agcgtggacc gcagcaagaa caccctgtac
ctgcagatga acagcctgcg cgccgaggac accgccgtgt actactgcgc ccgcaacctg ggccccagct tctacttcga
ctactggggc cagggcaccc tggtgaccgt gagcagc
SEQ ID NO :74 
gacatccaga tgacccagag ccccagcagc ctgagcgcca gcgtgggcga ccgcgtgacc atcacctgca aggccagcca
ggacgtgagc atcggcgtgg cctggtacca gcagaagccc ggcaaggccc ccaagtgcct gatctacagc gccagctacc
gctacaccgg cgtgcccagc cgcttcagcg gcagcggcag cggcaccgac ttcaccctga ccatcagcag cctgcagccc
gaggacttcg ccacctacta ctgccagcag tactacatct acccctacac cttcggccag ggcaccaagg tggagatcaa g
SEQ ID NO :75
SEQ ID NO :76 
DIQMTQSPSSLSASVGDRVTITCKASQDVSIGVAWYQQKPGKAPKCLIYSASYRYTGVPS
RFSGSGSGTDFTLTISSLQPEDFATYYCQQYYIYPYTFGQGTKVEIK
Pertuzumab scFv-87C
SEQ ID NO :77 
gacatccaga tgacccagag ccccagcagc ctgagcgcca gcgtgggcga ccgcgtgacc atcacctgca aggccagcca
ggacgtgagc atcggcgtgg cctggtacca gcagaagccc ggcaaggccc ccaagctgct gatctacagc gccagctacc
gctacaccgg cgtgcccagc cgcttcagcg gcagcggcag cggcaccgac ttcaccctga ccatcagcag cctgcagccc
gaggacttcg ccacctactg ctgccagcag tactacatct acccctacac cttcggccag ggcaccaagg tggagatcaa g
SEQ ID NO:78 
DIQMTQSPSSLSASVGDRVTITCKASQDVSIGVAWYQQKPGKAPKLLIYSASYRYTGVPS
RFSGSGSGTDFTLTISSLQPEDFATYCCQQYYIYPYTFGQGTKVEIK
Rit-46C
SEQ ID NO :79 
caagtgcagc tgcagcagcc tggcgccgag ctggtgaagc ctggcgctag cgtgaagatg agctgcaagg ctagcggcta
caccttcaca agctacaaca tgcactgggt gaagcagacc cctggcagag gcctggagtg gatcggcgcc atctaccctg
gcaacggcga cacaagctac aatcagaagt tcaagggcaa ggccaccctg accgccgaca agagcagcag caccgcctac
atgcagctga gcagcctgac aagcgaggac agcgccgtgt actactgcgc tagaagcacc tactacggcg gcgactggta
cttcaatgtg tggggagctg gcaccaccgt caccgtgagc gcc
SEQ ID NO :80 
caaatcgtgc tgagccaaag ccctgccatc ctgagcgcta gccctggcga gaaggtgacc atgacctgca gagctagcag
cagcgtgagc tacatccact ggtttcagca gaagcctggc agcagcccta agtgctggat ctacgccaca agcaacctgg
ctagcggcgt gcctgtgaga ttcagcggca gcggcagcgg cacaagctac agcctgacca tcagcagagt ggaggccgag
 gacgccgcca cctactactg tcagcagtgg acaagcaacc ctcctacctt cggcggaggc accaagctgg agatcaag
SEQ ID NO :81
SEQ ID NO :82 
QIVLSQSPAILSASPGEKVTMTCRASSSVSYIHWFQQKPGSSPKCWIYATSNLASGVPVRFS
GSGSGTSYSLTISRVEAEDAATYYCQQWTSNPPTFGGGTKLEIK
Mur-46C
SEQ ID NO :83 
caagtgcagc tgcagcagag cggcgccgag ctggctagac ctggcgctag cgtgaagatg agctgcaagg ctagcggcta
caccttcaca agatacacca tgcactgggt gaagcagaga cctggccaag gcctggagtg gatcggctac atcaacccta
gcagaggcta caccaactac aatcagaagt tcaaggacaa ggccaccctg accaccgaca agagcagcag caccgcctac
atgcagctga gcagcctgac aagcgaggac agcgccgtgt actactgcgc tagatactac gacgaccact actgcctgga
ctactggggc caaggcacca ccctgaccgt gagcagc
SEQ ID NO :84 
caaatcgtgc tgacacagag ccctgccatc atgagcgcta gccctggcga gaaggtgacc atgacctgca gcgctagcag
cagcgtgagc tacatgaact ggtatcagca gaagagcggc acaagcccta agtgctggat ctacgacaca agcaagctgg
ctagcggcgt gcctgcccac ttcagaggca gcggctccgg cacatcctac agcctgacca tcagcggcat ggaggccgag
gacgccgcca cctactactg tcagcagtgg agcagcaacc ctttcacctt cggcagcggc accaagctgg agatc
SEQ ID NO :85
SEQ ID NO :86 
QIVLTQSPAIMSASPGEKVTMTCSASSSVSYMNWYQQKSGTSPKCWIYDTSKLASGVPAH
FRGSGSGTSYSLTISGMEAEDAATYYCQQWSSNPFTFGSGTKLEI
Ram-87C
SEQ ID NO :87 
gaggtgcagc tggtgcagag cggcggcgga ctggtgaagc ctggcggcag cctgagactg agctgcgccg ctagcggctt
caccttcagc agctacagca tgaactgggt gagacaagcc cctggcaagg gcctggagtg ggtgagcagc atcagcagca
gcagcagcta catctactac gccgacagcg tgaagggcag attcaccatc agcagagaca acgccaagaa cagcctgtac
ctgcagatga acagcctgag agccgaggac accgccgtgt actactgcgc tagagtgacc gacgccttcg acatctgggg
 ccaaggcaca atggtgacag tcagcagc
SEQ ID NO :88 
gacattcaga tgacacagag ccctagcagc gtgagcgcta gcatcggcga cagagtgacc atcacctgca gagctagcca
aggcatcgac aactggctgg gctggtatca gcagaagcct ggcaaggccc ctaagctgct gatctacgac gctagcaacc
tggacaccgg cgtgcctagc agattcagcg gcagcggcag cggcacctac ttcaccctga ccatcagcag cctgcaagcc
gaggacttcg ccgtgtactg ctgtcagcaa gccaaggcct tccctcctac cttcggcgga ggcaccaagg tggacatcaa g
SEQ ID NO : 89
SEQ ID NO :90 
DIQMTQSPSSVSASIGDRVTITCRASQGIDNWLGWYQQKPGKAPKLLIYDASNLDTGVPS
RFSGSGSGTYFTLTISSLQAEDFAVYCCQQAKAFPPTFGGGTKVDIK
Per-VL-WT
SEQ ID NO :91 
gacattcaga tgacacagtc ccctagcagc ctgtccgcct ccgtgggcga cagagtgacc atcacctgta aggcctccca
agacgtgagc atcggcgtgg cttggtacca acaaaaacct ggaaaggccc ctaagctcct gatttacagc gcctcctata
gatacaccgg cgtgcctagc agattcagcg gaagcggcag cggcaccgac tttaccctga ccatcagcag cctgcagcct
gaggatttcg ctacctacta ttgccaacag tactacatct acccttacac cttcggccaa ggcaccaagg tggagatcaa g
SEQ ID NO :92
SEQ ID NO :93: ggaggaggag gcagc
SEQ ID NO :94: GGGGS
Rit-VL-WT
SEQ ID NO :95
Mur-VL-WT
SEQ ID NO :96 
QIVLTQSPAIMSASPGEKVTMTCSASSSVSYMNWYQQKSGTSPKRWIYDTSKLASGVPAH
FRGSGSGTSYSLTISGMEAEDAATYYCQQWSSNPFTFGSGTKLEI
Ram-VL-WT
SEQ ID NO :97
Trastuzumab-WT
SEQ ID NO :98 
GAAGTGCAACTGGTGGAGTCCGGAGGCGGACTGGTCCAACCCGGCGGCTCTCTGAGA
CTGAGCTGTGCTGCCAGCGGCTTCAACATCAAGGACACTTACATCCATTGGGTGAGG
CAAGCCCCCGGCAAAGGACTGGAATGGGTGGCTAGGATCTACCCAACTAACGGCTAC
ACTAGGTATGCCGACAGCGTCAAGGGAAGGTTCACTATCTCCGCCGACACAAGCAAG
AACACTGCCTATCTGCAGATGAACTCTCTGAGGGCTGAGGACACAGCTGTGTATTACT
GCTCTAGGTGGGGCGGAGATGGCTTCTACGCTATGGACTACTGGGGACAAGGCACAC
TCGTGACAGTGAGCAGCGCTAGCACTAAAGGCCCTTCCGTGTTCCCACTCGCCCCTAG
CTCCAAAAGCACTAGCGGCGGAACAGCTGCTCTGGGCTGTCTCGTGAAGGACTATTT
CCCAGAGCCAGTGACTGTGAGCTGGAATAGCGGCGCTCTGACAAGCGGAGTGCACAC
TTTTCCAGCCGTGCTGCAGAGCAGCGGACTGTATTCTCTGTCCAGCGTCGTCACTGTG
CCTAGCTCCTCTCTGGGCACACAGACTTACATCTGTAACGTGAATCATAAGCCAAGCA
ACACTAAGGTCGACAAGAAGGTCGAGCCAAAGAGCTGTGACAAGACACACACATGC
CCACCATGTCCAGCCCCAGAGCTCCTCGGAGGACCTAGCGTGTTCCTCTTCCCTCCTA
AGCCAAAGGACACTCTCATGATCTCTAGGACACCAGAGGTCACTTGCGTGGTGGTCG
ATGTCAGCCACGAGGACCCAGAGGTGAAGTTCAATTGGTACGTGGACGGCGTGGAGG
TGCATAATGCCAAGACAAAGCCTAGGGAGGAGCAGTACAACAGCACTTACAGAGTG
GTCTCCGTCCTCACAGTGCTGCACCAAGATTGGCTCAACGGCAAGGAGTACAAGTGC
AAGGTGTCCAACAAGGCCCTCCCAGCCCCTATCGAGAAGACTATCTCCAAGGCCAAG
GGCCAGCCAAGAGAACCTCAAGTGTACACACTGCCTCCATCTAGGGAGGAGATGACA
AAGAACCAAGTGTCTCTGACATGTCTGGTGAAAGGCTTCTACCCAAGCGACATCGCC
GTGGAGTGGGAAAGCAACGGCCAGCCAGAGAACAACTACAAGACTACACCTCCAGT
GCTGGACTCCGACGGCAGCTTCTTCCTCTACTCCAAGCTCACTGTGGATAAGTCTAGG
TGGCAGCAAGGCAATGTGTTCAGCTGTTCCGTGATGCACGAGGCTCTGCACAACCAC
TACACTCAGAAGTCTCTGAGCCTCTCCCCCGGCAAA
SEQ ID NO :99 
GACATCCAGATGACACAGTCCCCTTCCTCTCTGAGCGCTAGCGTCGGCGATAGGGTGA
CAATCACTTGTAGGGCCAGCCAAGATGTCAACACAGCCGTGGCTTGGTACCAGCAGAA
GCCCGGCAAGGCCCCTAAGCTGCTCATCTATAGCGCCAGCTTTCTGTACAGCGGCGTCC
 
CTAGCAGATTCTCCGGAAGCAGATCCGGCACTGACTTCACACTGACTATCAGCAGCCT
CCAGCCAGAGGATTTCGCCACATACTACTGCCAGCAGCACTACACTACTCCACCTACTT
TCGGCCAAGGCACTAAGGTGGAGATTAAGAGGACTGTCGCTGCCCCAAGCGTCTTCAT
CTTCCCACCTAGCGATGAACAGCTCAAGAGCGGCACAGCTAGCGTCGTGTGTCTGCTG
AACAACTTCTACCCTAGGGAGGCCAAGGTCCAGTGGAAAGTGGATAACGCTCTGCAG
AGCGGCAATTCCCAAGAGAGCGTCACAGAGCAAGATAGCAAAGACAGCACTTACTCT
CTGAGCAGCACACTGACTCTGTCCAAGGCCGACTACGAGAAGCACAAGGTCTATGCTT
GTGAGGTCACTCACCAAGGACTGTCCAGCCCAGTCACTAAGAGCTTCAATAGGGGCG
AGTGC
SEQ ID NO :100 
EVQLVESGGGLVQPGGSLRLSCAASGFNIKDTYIHWVRQAPGKGLEWVARIYPTNGYTR
YADSVKGRFTISADTSKNTAYLQMNSLRAEDTAVYYCSRWGGDGFYAMDYWGQGTLV
TVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPS
REE
MTKNQVSLTCLVKGFYPSDIAVEWESNGQPENNYKTTPPVLDSDGSFFLYSKLTVDKSR
WQQGNVFSCSVMHEALHNHYTQKSLSLSPGK
SEQ ID NO :101 
DIQMTQSPSSLSASVGDRVTITCRASQDVNTAVAWYQQKPGKAPKLLIYSASFLYSGVPS
RFSGSRSGTDFTLTISSLQPEDFATYYCQQHYTTPPTFGQGTKVEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO : 102 (the coding sequence of 46C mutant) 
GACATCCAGATGACACAGTCCCCTTCCTCTCTGAGCGCTAGCGTCGGCGATAGGGTGA
CAATCACTTGTAGGGCCAGCCAAGATGTCAACACAGCCGTGGCTTGGTACCAGCAGAA
GCCCGGCAAGGCCCCTAAGTGTCTCATCTATAGCGCCAGCTTTCTGTACAGCGGCGTC
CCTAGCAGATTCTCCGGAAGCAGATCCGGCACTGACTTCACACTGACTATCAGCAGCC
TCCAGCCAGAGGATTTCGCCACATACTACTGCCAGCAGCACTACACTACTCCACCTACT
TTCGGCCAAGGCACTAAGGTGGAGATTAAGAGGACTGTCGCTGCCCCAAGCGTCTTCA
TCTTCCCACCTAGCGATGAACAGCTCAAGAGCGGCACAGCTAGCGTCGTGTGTCTGCT
GAACAACTTCTACCCTAGGGAGGCCAAGGTCCAGTGGAAAGTGGATAACGCTCTGCA
GAGCGGCAATTCCCAAGAGAGCGTCACAGAGCAAGATAGCAAAGACAGCACTTACTC
 
TCTGAGCAGCACACTGACTCTGTCCAAGGCCGACTACGAGAAGCACAAGGTCTATGCT
TGTGAGGTCACTCACCAAGGACTGTCCAGCCCAGTCACTAAGAGCTTCAATAGGGGCG
AGTGC
SEQ ID NO : 103 (the amino acid sequence of 46C mutant) 
DIQMTQSPSSLSASVGDRVTITCRASQDVNTAVAWYQQKPGKAPKCLIYSASFLYSGVPS
RFSGSRSGTDFTLTISSLQPEDFATYYCQQHYTTPPTFGQGTKVEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO :104 (the coding sequence of 87C mutant) 
GACATCCAGATGACACAGTCCCCTTCCTCTCTGAGCGCTAGCGTCGGCGATAGGGTGA
CAATCACTTGTAGGGCCAGCCAAGATGTCAACACAGCCGTGGCTTGGTACCAGCAGAA
GCCCGGCAAGGCCCCTAAGCTGCTCATCTATAGCGCCAGCTTTCTGTACAGCGGCGTCC
CTAGCAGATTCTCCGGAAGCAGATCCGGCACTGACTTCACACTGACTATCAGCAGCCT
CCAGCCAGAGGATTTCGCCACATACTGTTGCCAGCAGCACTACACTACTCCACCTACTT
TCGGCCAAGGCACTAAGGTGGAGATTAAGAGGACTGTCGCTGCCCCAAGCGTCTTCAT
CTTCCCACCTAGCGATGAACAGCTCAAGAGCGGCACAGCTAGCGTCGTGTGTCTGCTG
AACAACTTCTACCCTAGGGAGGCCAAGGTCCAGTGGAAAGTGGATAACGCTCTGCAG
AGCGGCAATTCCCAAGAGAGCGTCACAGAGCAAGATAGCAAAGACAGCACTTACTCT
CTGAGCAGCACACTGACTCTGTCCAAGGCCGACTACGAGAAGCACAAGGTCTATGCTT
GTGAGGTCACTCACCAAGGACTGTCCAGCCCAGTCACTAAGAGCTTCAATAGGGGCG
AGTGC
SEQ ID NO :105 (the amino acid sequence of 87C mutant) 
DIQMTQSPSSLSASVGDRVTITCRASQDVNTAVAWYQQKPGKAPKLLIYSASFLYSGVPS
RFSGSRSGTDFTLTISSLQPEDFATYCCQQHYTTPPTFGQGTKVEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO:106 
CAGGTGCAGCTGAAGCAGAGCGGCCCCGGCCTGGTGCAGCCCAGCCAGAGCCTGAGC
ATCACCTGCACCGTGAGCGGCTTCAGCCTGACCAACTACGGCGTGCACTGGGTGAGG
CAGAGCCCCGGCAAGGGCCTGGAGTGGCTGGGCGTGATCTGGAGCGGCGGCAACAC
CGACTACAACACCCCCTTCACCAGCAGGCTGAGCATCAACAAGGACAACAGCAAGAG
CCAGGTGTTCTTCAAGATGAACAGCCTGCAGAGCAACGACACCGCCATCTACTACTG
CGCCAGGGCCCTGACCTACTACGACTACGAGTTCGCCTACTGGGGCCAGGGCACCCT
 GGTGACCGTGAGCGCC
SEQ ID NO :107 
GACATCCTGCTGACCCAGAGCCCCGTGATCCTGAGCGTGAGCCCCGGCGAGAGGGTG
AGCTTCAGCTGCAGGGCCAGCCAGAGCATCGGCACCAACATCCACTGGTACCAGCAG
AGGACCAACGGCAGCCCCAGGCTGCTGATCAAGTACGCCAGCGAGAGCATCAGCGG
CATCCCCAGCAGGTTCAGCGGCAGCGGCAGCGGCACCGACTTCACCCTGAGCATCAA
CAGCGTGGAGAGCGAGGACATCGCCGACTACTACTGCCAGCAGAACAACAACTGGCC
CACCACCTTCGGCGCCGGCACCAAGCTGGAGCTGAAG
SEQ ID NO :108 
QVQLKQSGPGLVQPSQSLSITCTVSGFSLTNYGVHWVRQSPGKGLEWLGVIWSGGNTDY
NTPFTSRLSINKDNSKSQVFFKMNSLQSNDTAIYYCARALTYYDYEFAYWGQGTLVTVSA
SEQ ID NO :109 
DILLTQSPVILSVSPGERVSFSCRASQSIGTNIHWYQQRTNGSPRLLIKYASESISGIPSRFSGS
GSGTDFTLSINSVESEDIADYYCQQNNNWPTTFGAGTKLELK
SEQ ID NO:110 
CAGGTGCAGCTGGTGCAGAGCGGCGCCGAGGTGAAGAAGCCCGGCGCCAGCGTGAA
GGTGAGCTGCAAGGCCAGCGGCTACACCTTCACCAGCTACGGCTTCAGCTGGGTGAG
GCAGGCCCCCGGCCAGGGCCTGGAGTGGATGGGCTGGATCAGCGCCAGCAACGGCAA
CACCTACTACGCCCAGAAGCTGCAGGGCAGGGTGACCATGACCACCGACACCAGCAC
CAGCACCGCCTACATGGAGCTGAGGAGCCTGAGGAGCGACGACACCGCCGTGTACTA
CTGCGCCAGGGTGTACGCCGACTACGCCGACTACTGGGGCCAGGGCACCCTGGTGAC
CGTGAGCAGC
SEQ ID NO:111 
GACATCCAGATGACCCAGAGCCCCAGCAGCGTGAGCGCCAGCGTGGGCGACAGGGTG
ACCATCACCTGCAGGGCCAGCCAGGGCATCAACACCTGGCTGGCCTGGTACCAGCAG
AAGCCCGGCAAGGCCCCCAAGCTGCTGATCTACGCCGCCAGCAGCCTGAAGAGCGGC
GTGCCCAGCAGGTTCAGCGGCAGCGGCAGCGGCACCGACTTCACCCTGACCATCAGC
AGCCTGCAGCCCGAGGACTTCGCCACCTACTACTGCCAGCAGGCCAACAGCTTCCCCC
TGACCTTCGGCGGCGGCACCAAGGTGGAGATCAAG
SEQ ID NO:112
   GTGGAGGGCGGCAGCGGCGGCAGCGGCGGCAGCGGCGGCAGCGGCGGCGTGGAC
SEQ ID NO:113 
QVQLVQSGAEVKKPGASVKVSCKASGYTFTSYGFSWVRQAPGQGLEWMGWISASNGNT
YYAQKLQGRVTMTTDTSTSTAYMELRSLRSDDTAVYYCARVYADYADYWGQGTLVTVSS
SEQ ID NO:114 
DIQMTQSPSSVSASVGDRVTITCRASQGINTWLAWYQQKPGKAPKLLIYAASSLKSGVPSR
FSGSGSGTDFTLTISSLQPEDFATYYCQQANSFPLTFGGGTKVEIK
SEQ ID NO:115
   VEGGSGGSGGSGGSGGVD
SEQ ID NO:116 
GACATCCTGCTGACCCAGAGCCCCGTGATCCTGAGCGTGAGCCCCGGCGAGAGGGTG
AGCTTCAGCTGCAGGGCCAGCCAGAGCATCGGCACCAACATCCACTGGTACCAGCAG
AGGACCAACGGCAGCCCCAGGtgcCTGATCAAGTACGCCAGCGAGAGCATCAGCGGCA
TCCCCAGCAGGTTCAGCGGCAGCGGCAGCGGCACCGACTTCACCCTGAGCATCAACA
GCGTGGAGAGCGAGGACATCGCCGACTACTACTGCCAGCAGAACAACAACTGGCCCA
CCACCTTCGGCGCCGGCACCAAGCTGGAGCTGAAG
SEQ ID NO:117 
DILLTQSPVILSVSPGERVSFSCRASQSIGTNIHWYQQRTNGSPRCLIKYASESISGIPSRFSG
SGSGTDFTLSINSVESEDIADYYCQQNNNWPTTFGAGTKLELK
SEQ ID NO:118 
GACATCCAGATGACCCAGAGCCCCAGCAGCGTGAGCGCCAGCGTGGGCGACAGGGT
GACCATCACCTGCAGGGCCAGCCAGGGCATCAACACCTGGCTGGCCTGGTACCAGCA
GAAGCCCGGCAAGGCCCCCAAGtgcCTGATCTACGCCGCCAGCAGCCTGAAGAGCGGC
GTGCCCAGCAGGTTCAGCGGCAGCGGCAGCGGCACCGACTTCACCCTGACCATCAGC
AGCCTGCAGCCCGAGGACTTCGCCACCTACTACTGCCAGCAGGCCAACAGCTTCCCC
CTGACCTTCGGCGGCGGCACCAAGGTGGAGATCAAG
SEQ ID NO:119 
DIQMTQSPSSVSASVGDRVTITCRASQGINTWLAWYQQKPGKAPKCLIYAASSLKSGVPS
RFSGSGSGTDFTLTISSLQPEDFATYYCQQANSFPLTFGGGTKVEIK
SEQ ID NO:120 
GACATCCAGATGACCCAGAGCCCCAGCAGCGTGAGCGCCAGCGTGGGCGACAGGGT
GACCATCACCTGCAGGGCCAGCCAGGGCATCAACACCTGGCTGGCCTGGTACCAGCA
GAAGCCCGGCAAGGCCCCCAAGCTGCTGATCTACGCCGCCAGCAGCCTGAAGAGCGG
CGTGCCCAGCAGGTTCAGCGGCAGCGGCAGCGGCACCGACTTCACCCTGACCATCAG
CAGCCTGCAGCCCGAGGACTTCGCCACCTACtgcTGCCAGCAGGCCAACAGCTTCCCCC
TGACCTTCGGCGGCGGCACCAAGGTGGAGATCAAG
SEQ ID NO:121 DIQMTQSPSSVSASVGDRVTITCRASQGINTWLAWYQQKPGKAPKLLIYAASSLKSGVPS RFSGSGSGTDFTLTISSLQPEDFATYCCQQANSFPLTFGGGTKVEIK
SEQ ID NO:122 
GACATCCTGCTGACCCAGAGCCCCGTGATCCTGAGCGTGAGCCCCGGCGAGAGGGTG
AGCTTCAGCTGCAGGGCCAGCCAGAGCATCGGCACCAACATCCACTGGTACCAGCAG
AGGACCAACGGCAGCCCCAGGCTGCTGATCAAGTACGCCAGCGAGAGCATCAGCGG
CATCCCCAGCAGGTTCAGCGGCAGCGGCAGCGGCACCGACTTCACCCTGAGCATCAA
CAGCGTGGAGAGCGAGGACATCGCCGACTACTGCTGCCAGCAGAACAACAACTGGCC
CACCACCTTCGGCGCCGGCACCAAGCTGGAGCTGAAG
SEQ ID NO:123 
DILLTQSPVILSVSPGERVSFSCRASQSIGTNIHWYQQRTNGSPRLLIKYASESISGIPSRFSG
SGSGTDFTLSINSVESEDIADYCCQQNNNWPTTFGAGTK
SEQ ID NO:124
   GGCGGGGGCGGGTCCGGCGGCGGGGGCAGC
SEQ ID NO:125
   GGGGSGGGGS
SEQ ID NO:126 
GGCGGGGGCGGGTCCGGCGGCGGGGGCAGCGGGGGCGGGGGGAGCGGCGGGGGGG
GCAGC
SEQ ID NO:127
   GGGGSGGGGSGGGGSGGGGSGGGGS
SEQ ID NO: 128 (the coding sequence of the heavy chain variable region of Zalutumumab) 
CAAGTGCAGCTGGTGGAATCTGGCGGAGGTGTTGTGCAGCCTGGCAGAAGCCTGAGA
CTGTCCTGCGCCGCTAGCGGCTTCACCTTCAGCACCTACGGCATGCACTGGGTCAGAC
AGGCCCCTGGAAAAGGCCTGGAGTGGGTGGCCGTGATCTGGGACGACGGCTCTTATA
AGTACTACGGCGACAGCGTTAAGGGCAGGTTCACCATCAGCAGAGATAATAGCAAGA
ACACCCTGTACCTGCAGATGAACAGCCTGCGGGCCGAGGACACTGCCGTGTACTACT
GTGCCAGAGATGGCATCACAATGGTGCGGGGCGTGATGAAAGACTACTTCGACTACT
GGGGCCAGGGCACACTGGTGACAGTGTCCTCC
SEQ ID NO: 129 (the coding sequence of the light chain variable region of Zalutumumab LC46C) 
GCTATCCAGCTGACCCAGAGCCCAAGCAGCCTGAGCGCCTCTGTGGGCGATAGAGTG
ACCATTACATGCAGAGCCTCCCAGGACATCTCTAGTGCTCTGGTGTGGTACCAGCAAA
AGCCTGGCAAGGCCCCTAAGTGCCTGATCTACGACGCCAGCAGCCTGGAAAGCGGAG
TGCCTAGCCGGTTTAGCGGCAGCGAGAGCGGCACCGACTTCACCCTGACCATCAGCT
CTCTCCAGCCCGAGGATTTTGCCACCTATTACTGCCAGCAGTTCAACAGCTACCCCCT
GACATTCGGCGGAGGAACCAAGGTGGAAATCAAG
SEQ ID NO: 130 (the amino acid sequence of the heavy chain variable region of Zalutumumab)
SEQ ID NO:131 (the amino acid sequence of the light chain variable region of Zalutumumab LC46C) 
AIQLTQSPSSLSASVGDRVTITCRASQDISSALVWYQQKPGKAPKCLIYDASSLESGVPSRF
SGSESGTDFTLTISSLQPEDFATYYCQQFNSYPLTFGGGTKVEIK
SEQ ID NO: 132 (the coding sequence of the light chain variable region of Zalutumumab) 
GCTATCCAGCTGACCCAGAGCCCAAGCAGCCTGAGCGCCTCTGTGGGCGATAGAGTGA
CCATTACATGCAGAGCCTCCCAGGACATCTCTAGTGCTCTGGTGTGGTACCAGCAAAA
GCCTGGCAAGGCCCCTAAGCTGCTGATCTACGACGCCAGCAGCCTGGAAAGCGGAGT
GCCTAGCCGGTTTAGCGGCAGCGAGAGCGGCACCGACTTCACCCTGACCATCAGCTCT
CTCCAGCCCGAGGATTTTGCCACCTATTACTGCCAGCAGTTCAACAGCTACCCCCTGAC
ATTCGGCGGAGGAACCAAGGTGGAAATCAAG
SEQ ID NO: 133 (the amino acid sequence of the light chain variable region of Zalutumumab)

## Claims

1. A cysteine-engineered antibody, wherein, according to the Kabat numbering system, the antibody has an engineered cysteine residue at any one or more position(s) selected from:
positions 12, 34, 35, 38, 44, 47, 51, 60, 61, 67, 69, 78, 79, and 114 of the heavy chain variable region, or any combination thereof; or
positions 19, 21, 44, 46, 47, 48, 62, 71, 75, 78, and 87 of the light chain variable region, or any combination thereof.

2. The cysteine-engineered antibody according to claim 1, wherein, according to the Kabat numbering system, the antibody has an engineered cysteine residue at any one or more position(s) selected from:
positions 12, 35, 61, 51, 69, and 78 of the heavy chain variable region, or any combination thereof; or
positions 46, and 87 of the light chain variable region, or any combination thereof.

3. The cysteine-engineered antibody according to claim 1, wherein, according to the Kabat numbering system, the antibody has an engineered cysteine residue at any one or more positions selected from:
positions 12, 35, 61, and 51 of the heavy chain variable region, or any combination thereof; or
positions 46, and 87 of the light chain variable region, or any combination thereof.

4. The cysteine-engineered antibody according to claim 1, wherein, the cysteine-engineered antibody is derived from the following wild-type antibody or antibodies:
an antibody comprising the heavy chain variable region as set forth in SEQ ID NO: 4 and the light chain variable region as set forth in SEQ ID NO: 5;
an antibody comprising the heavy chain variable region as set forth in SEQ ID NO: 75 and the light chain variable region as set forth in SEQ ID NO: 92;
an antibody comprising the heavy chain variable region as set forth in SEQ ID NO: 81 and the light chain variable region as set forth in SEQ ID NO: 95;
an antibody comprising the heavy chain variable region as set forth in SEQ ID NO: 85 and the light chain variable region as set forth in SEQ ID NO: 96;
an antibody comprising the heavy chain variable region as set forth in SEQ ID NO: 89 and the light chain variable region as set forth in SEQ ID NO: 97;
an antibody comprising the heavy chain as set forth in SEQ ID NO: 100 and the light chain as set forth in SEQ ID NO: 101;
an antibody comprising the heavy chain variable region as set forth in SEQ ID NO: 108 and the light chain variable region as set forth in SEQ ID NO: 109;
an antibody comprising the heavy chain variable region as set forth in SEQ ID NO: 113 and the light chain variable region as set forth in SEQ ID NO: 114; and/or
an antibody comprising the heavy chain variable region as set forth in SEQ ID NO: 130 and the light chain variable region as set forth in SEQ ID NO: 133.

5. The cysteine-engineered antibody according to claim 1, wherein, the light chain of the antibody is type λ or κ;
optionally, the antibody is an anti-HER2 antibody, an anti-CD3 antibody, an anti-CD20 antibody, an anti-VEGFR-2 antibody, an anti-EGFR antibody, and/or an anti-c-Met antibody;
optionally, the antibody is a single chain antibody fragment, an IgG antibody, or a bispecific antibody such as a bispecific antibody in the form of BiTE/DART/Diabody.

6. The cysteine-engineered antibody according to claim 5, wherein, when the antibody is a single chain antibody fragment, the antibody further comprises a linking chain, preferably a linking chain of SEQ ID NO. 6.

7. A conjugate comprising the cysteine-engineered antibody according to any one of claims 1-6, and a conjugated moiety.

8. The conjugate according to claim 7, wherein,
the conjugated moiety is selected from: polyethylene glycol, a cytotoxic agent, an active peptide, a nanobody, a single domain antibody, a Fab fragment, a Fab' fragment, scFv, a small molecule drug (*e.g.*, a topoenzyme inhibitor, tubulysin A, DM1, PBD, MMAE, or MMAF, etc.), a chemotherapeutic agent, or a radiotherapeutic agent;
the conjugated moiety is conjugated to the cysteine-engineered antibody via a linker;
preferably, the linker comprises an electrophilic group (preferably a maleimide group or a haloacetamide group); optionally, the linker is mc-VC-PAB.

9. The conjugate according to claim 8, wherein, the polyethylene glycol is a Methoxy PEG Maleimide, preferably mPEG2000-Mal, mPEG5000-Mal, or mPEG10000-Mal.

10. A pharmaceutical composition comprising the cysteine-engineered antibody according to any one of claims 1-6 or the conjugate according to any one of claims 7-9, and optionally, a pharmaceutical carrier.

11. Use of the cysteine-engineered antibody according to any one of claims 1-6, the conjugate according to any one of claims 7-9, or the pharmaceutical composition according to claim 10 in the manufacture of a medicament or a kit for the treatment of a cancer (*e.g.*, a HER2-positive cancer, preferably breast cancer).

12. A kit comprising the cysteine-engineered antibody according to any one of claims 1-6, the conjugate according to any one of claims 7-9, or the pharmaceutical composition according to claim 10.
